# EUROPEAN PATENT SPECIFICATION

(11) **EP 3 883 538 B1**
(45) Date of publication and mention of the grant of the patent: **21.08.2024**
(21) Application number: 19836902.7
(22) Date of filing: 17.12.2019
(51) Int. Cl.: A61K 8/44, A61Q 11/02, A61Q 11/00

(54) **REDUCTION OF PATHOGENIC BACTERIA USING ARGININE**
VERMINDERUNG PATHOGENER BAKTERIEN MIT ARGININ
RÉDUCTION DES BACTÉRIES PATHOGÈNES GRÂCE À L'ARGININE

(30) Priority: 26.12.2018 US 201862784945 P
(43) Date of publication of application: 29.09.2021
(73) Proprietor: Colgate-Palmolive Company, New York, NY 10022 (US)
(72) Inventor: DAEP, Carlo, Avenel, NJ 07001 (US); MAKWANA, Ekta, Monroe, New Jersey 08831 (US); ZAIDEL, Lynette, Cranford, New Jersey 07016 (US)
(74) Representative: Sonnenhauser, Thomas Martin
(86) International application number: PCT/US2019/066800
(87) International publication number: WO 2020/139619

(56) References cited:
- WO-A1-2015/048146

## Description

### BACKGROUND

Current studies within the gut microbiome have reflected potential benefits of beneficial bacteria to overall health. Promoting the growth of select bacteria (e.g. *Bifidobacteria* and *Lactobacilli* sp) have been observed to have important benefits on gastrointestinal health and a person's overall well-being. One avenue that is currently being pursued in the field of gut microbiology is prebiotics. Prebiotics are defined as non-digestible substances that induce the growth or bacteria that can contribute to improving host health. These compounds serve as an important selective nutritional tool that can effectively alter the composition of the microbiom, improving the overall health status of the gut microbiota and gut health. This suggests that influencing the microbial community within the host may have important implications in promoting better health. Interestingly, the approach has not yet been applied to the oral microbiome to improve oral health.

The oral microbial community is comprised of ~ 700 different microbial species including beneficial oral bacteria species and pathogenic oral bacteria species.

Beneficial bacteria within the oral microflora appear to play an important role in health, producing factors that are associated with oral health. Beneficial bacteria may, by their presence or metabolic activity, cause in one or more of the following effects: lowering the number or proportion of pathogenic oral bacteria; lowering inflammation and inflammatory processes; lowering the metabolic activity of pathogenic oral bacteria species; lowering the production or inhibiting virulence factors produced by pathogenic oral bacteria species; lowering or inhibiting biofilm formation; occupying a niche which may otherwise be colonized by pathogens; limiting a pathogen's ability to adhere to oral surfaces; affecting the viability, metabolic activity or growth of a pathogen; lowering the ability of a pathogen to produce virulence factors; degrading virulence factors produced by the pathogen or the oral microbiota; and/or attenuating the host response to pathogens. Certain species of oral bacteria may be beneficial for maintaining the health of the periodontium. Without being bound by any theory, it is believed that beneficial oral bacteria can interfere with colonization by pathogenic oral bacteria of the oral epithelium and in biofilm in the oral cavity. For example, studies have shown that *Streptococcus sanguinis, Streptococcus mitis* and *Streptococcus salivarius* have inhibitory effects on *A. actinomycetemcomitans* colonization of epithelial cells *in vitro* (W. Teughels et al., J Dent Res 86(7), 611-617, 2007). It has also been shown, using a canine model, that the application of beneficial bacteria to periodontal pockets following root planing delays and reduces recolonization of the periodontal pockets by pathogenic bacteria (W. Teughels, et al., J Dent Res, 86(11), 1078-1082, 2007). The beneficial bacteria *S. sanguinis, S. mitis* and *S. salivarius* have also been shown to inhibit *Aggregatibacter actinomycetemcomitans-induced* production of the inflammatory cytokine interleukin-8 (IL-8) by the human oral keratinocyte cell line HOK-18A, which inflammatory response is implicated in periodontitis-related tissue destruction (I. Sliepen et al., J Dent Res 88(11), 1026-1030, 2009).

Pathogenic bacteria species are associated with diseases and disorders. Some species of oral pathogenic bacteria (e.g. *Porphyromonas gingivalis, Tannerella forsythia* and *A. actinomycetemcomitans)* have been implicated in the development of periodontal diseases, such as periodontitis, gingivitis, necrotizing periodontitis, necrotizing gingivitis and peri-implantitis. Certain species of oral pathogenic bacteria have been implicated in tooth decay (e.g. *Streptococcus mutans*)*.* The onset of such oral diseases and conditions is largely due to the populational increase in pathogenic bacteria either on the tooth surface (cariogenic bacteria) or within the sub-gingiva (periodontal pathogens).

Current approaches to control such oral diseases and conditions is either through the reduction of dental plaque to improve gum health or the use of chemical agents (e.g. fluoride) to facilitate enamel remineralization and circumvent the deleterious effects of cariogenic bacteria. Compositions and methods that include use of standard anti-bacterial therapeutic agents are designed to promote good oral health by eliminating pathogenic bacteria. Since the microbial community in the oral cavity also harbors beneficial bacteria, beneficial bacteria may also be eliminated by the use of standard anti-bacterial therapeutic agents. Therefore, a non-selective approach in reducing oral plaque may not be an optimal approach in promoting oral health.

Selectively promoting the growth of beneficial oral bacteria may provide a valid preventative approach for oral health, such as for example in the prevention of periodontitis and/or prevention of cavities. Methods of promoting beneficial bacteria in the oral cavity change plaque composition towards a healthier state with less pathogens. Compositions and methods that selectively promote the growth of beneficial oral bacteria relative to the growth of pathogenic bacteria in the oral cavity, such as for example in biofilm in the oral cavity may reduce total pathogenic oral bacteria load within the oral cavity. Compositions and methods that selectively promote the growth of beneficial oral bacteria relative to the growth of pathogenic bacteria in the oral cavity, such as for example in biofilm in the oral cavity, may promote good oral health and prevent or reduce the severity of diseases and disorders caused by pathogenic bacteria. Moreover, methods to identify compounds and compositions that selectively promote the growth of beneficial oral bacteria relative to the growth of pathogenic bacteria in the oral cavity, such as for example in biofilm in the oral cavity and methods of identifying compositions that promote changing plaque composition towards a healthier state with less pathogens are useful processes to identify compounds and compositions that promote good oral health. WO 2015/048146 A1 discloses compositions and methods for destabilizing biofilms, altering biofilm 3D structure, and dispersing biofilms, in order to enhance biofilm cell removal and/or sensitivity to other agents (e.g., environmental or co-applied treatments) and relates to the use of L-arginine in the removal and/or sensitization (e.g., to antimicrobials) of microorganisms in medical, industrial, domestic, or environmental applications, as well as treatment of bacterial infections (e.g., in biofilms).

### BRIEF SUMMARY

Methods of selectively promoting beneficial oral bacteria and reducing total pathogenic oral bacteria load within an individual's oral cavity are useful in to change plaque composition towards a healthier state with less pathogens.

Claimed is an oral care composition comprising arginine for use in a method of reducing total pathogenic oral bacteria load within an individual's oral cavity wherein pathogenic oral bacteria are *Porphyromonas gingivalis, Prevotella intermedia, Streptococcus mutans* and *Streptococcus sobrinus,* and the method comprises the step of applying 1-50 mg of arginine to the oral cavity.. The methods comprise the step of applying 1-50 mg of arginine to the oral cavity.

The methods can comprise the step of applying to the oral cavity an amount of arginine sufficient to obtain a concentration of at least 20-25 micromol/ml or higher in the oral cavity.

The compositions may selectively promote growth of beneficial oral bacteria relative to growth of pathogenic oral bacteria in an individual's oral cavity. The methods comprise the step of applying 1-50 mg of arginine to the oral cavity.

The compositions may selectively promote growth of beneficial oral bacteria relative to growth of pathogenic oral bacteria in biofilm in an individual's oral. The methods comprise the step of applying 1-50 mg of arginine to the oral cavity.

The compositions may selectively promote growth of beneficial oral bacteria relative to growth of pathogenic oral bacteria in an individual's oral cavity. The methods may comprise the step of applying to the oral cavity an amount of arginine sufficient to obtain a concentration of at least 20-25 micromol/ml or higher in the oral cavity.

The compositions may selectively promote growth of beneficial oral bacteria relative to growth of pathogenic oral bacteria in biofilm in an individual's oral cavity. The methods may comprise the step of applying to the oral cavity an amount of arginine sufficient to obtain a concentration of at least 20-25 micromol/ml or higher in the oral cavity.

The compositions may selectively promote growth of beneficial oral bacteria relative to growth of pathogenic oral bacteria in a population of bacteria that comprises beneficial oral bacteria and pathogenic oral bacteria. The methods may comprise the step of contacting the population of bacteria with arginine at a concentration of at least 20-25 micromol/ml or higher.

The compositions may selectively promote growth of beneficial oral bacteria relative to growth of pathogenic oral bacteria in biofilm that comprises beneficial oral bacteria and pathogenic oral bacteria. The methods may comprise the step of contacting the biofilm that comprises beneficial oral bacteria and pathogenic oral bacteria with arginine at a concentration of at least 20-25 micromol/ml or higher.

Methods of identifying a compound or composition that enhances the selective promotion of growth of beneficial oral bacteria relative to growth of pathogenic oral bacteria by arginine can be used. The methods comprise the steps of: performing: a beneficial oral bacteria growth test assay; a pathogenic oral bacteria growth test assay; a beneficial oral bacteria growth control assay; and a pathogenic oral bacteria growth control assay. The beneficial oral bacteria growth test assay comprises culturing a species of beneficial oral bacteria in media that comprises arginine and a test compound or composition and measuring the growth of the beneficial oral bacteria in the beneficial oral bacteria growth test assay. The pathogenic oral bacteria growth test assay comprises culturing a species of pathogenic oral bacteria in media that comprises arginine and the test compound or composition and measuring the growth of the pathogenic oral bacteria in the pathogenic oral bacteria growth test assay. The c beneficial oral bacteria growth control assay comprises culturing a species of beneficial oral bacteria in media that comprises arginine free of the test compound or composition and measuring the growth of the beneficial oral bacteria in the beneficial oral bacteria growth control assay. The pathogenic oral bacteria growth control assay comprises culturing a species of pathogenic oral bacteria in media that comprises arginine free the test compound or composition and measuring the growth of the pathogenic oral bacteria in the pathogenic oral bacteria growth control assay. The ratio of the growth of the beneficial oral bacteria in the beneficial oral bacteria growth test assay to the growth of the beneficial oral bacteria in the beneficial oral bacteria growth control assay is calculated and the ratio of the growth of the pathogenic oral bacteria in the pathogenic oral bacteria growth test assay to the growth of the pathogenic oral bacteria in the pathogenic oral bacteria growth control assay is calculated. The ratios are compared. A ratio of the growth of the beneficial oral bacteria in the beneficial oral bacteria growth test assay to the growth of the beneficial oral bacteria in the beneficial oral bacteria growth control assay that is higher than the ratio of the growth of the pathogenic oral bacteria in the pathogenic oral bacteria growth test assay to the growth of the pathogenic oral bacteria in the pathogenic oral bacteria growth control indicates that the test compound or composition enhances the selective promotion of growth of beneficial oral bacteria relative to growth of pathogenic oral bacteria by arginine.

Methods of identifying a compound or composition that enhances the selective promotion of growth of beneficial oral bacteria relative to growth of pathogenic oral bacteria in biofilm by arginine can be used. The methods comprise the steps of performing a dual species biofilm test assay and a dual species biofilm control assay. The dual species biofilm test assay comprises: co-culturing a species of beneficial oral bacteria and a species of pathogenic oral bacteria in a dual species test biofilm that comprises arginine and a test compound or composition; quantifying beneficial oral bacteria and pathogenic oral bacteria in the dual species test biofilm; and comparing the quantity of beneficial oral bacteria to the quantity of pathogenic oral bacteria in the dual species test biofilm to determine the dual species test biofilm's beneficial oral bacteria-to-pathogenic oral bacteria load. The dual species biofilm control assay comprises: co-culturing a species of beneficial oral bacteria and a species of pathogenic oral bacteria in a dual species control biofilm that comprises arginine free of the test compound or composition; quantifying beneficial oral bacteria and pathogenic oral bacteria in the dual species control biofilm; and comparing the quantity of beneficial oral bacteria to the quantity of pathogenic oral bacteria in the dual species control biofilm to determine the dual species control biofilm's beneficial oral bacteria-to-pathogenic oral bacteria load. The dual species test biofilm's beneficial oral bacteria-to-pathogenic oral bacteria load is compared to the dual species control biofilm's beneficial oral bacteria-to-pathogenic oral bacteria load. A higher beneficial oral bacteria-to-pathogenic oral bacteria load in the dual species test biofilm compared to the beneficial oral bacteria-to-pathogenic oral bacteria load in the dual species control biofilm indicates that the test compound or composition enhances the selective promotion of growth of beneficial oral bacteria relative to growth of pathogenic oral bacteria in biofilm by arginine.

### DETAILED DESCRIPTION

As used herein, the expression "oral cavity" includes not only the cavity itself but also the saliva, teeth, gingiva, periodontal pockets, cheeks, tongue, mucosa, tonsils, any implants, and any device or structure which is placed into the oral cavity. The tonsils provide a reservoir (tonsil stones) for growth of anaerobic bacteria which may generate bad breath.

As used herein, the phrase "a healthy oral microbiota" refers to the microbial population of the oral cavity when the oral cavity is in a non-diseased state (for example, when there is no periodontal disease present, e.g. gingivitis, periodontitis, caries, peri-implantitis, peri-implant mucositis, necrotizing gingivitis and/or necrotizing periodontitis), i.e. a health-associated oral microbiota.

The term "beneficial oral bacteria" encompasses those bacteria which are present in the oral cavity in higher numbers or proportions in a healthy oral cavity, but which are present in lower numbers or proportions in conditions of oral disease (such as, for example, gingivitis, periodontitis, caries, peri-implantitis, peri-implant mucositis, necrotizing gingivitis and necrotizing periodontitis). This term also includes bacteria from oral or non-oral origins which have proven beneficial effects on oral health by preventing or treating oral diseases, which may be already present in the oral cavity or may be intentionally introduced into the oral cavity (for example as probiotics). Beneficial bacteria may, by their presence or metabolic activity, result in: lowering the number or proportion of pathogenic oral bacteria; lowering inflammation and inflammatory processes; lowering the metabolic activity of pathogenic species; lowering the production or inhibiting virulence factors produced by pathogenic bacteria; lowering or inhibiting biofilm formation; occupying a niche which may otherwise be colonized by pathogens; limiting a pathogen's ability to adhere to oral surfaces; affecting the viability, metabolic activity or growth of a pathogen; lowering the ability of a pathogen to produce virulence factors; degrading virulence factors produced by the pathogen or the oral microbiota; and/or attenuating the host response to pathogenic species. Examples of beneficial oral bacteria include *Streptococcus gordonii, Actinomyces viscosus, Streptococcus salivarius, Streptococcus oralis, Streptococcus mitis, Streptococcus sanguinis, Veillonella parvula, Capnocytophaga sputigena* and *Actinomyces naeslundii.*

Examples of pathogenic oral bacteria include *Streptococcus mutans, Prevotella intermedia, Porphyromonas gingivalis, Fusobacterium nucleatum, Tannerella forsythia, Aggregatibacter actinomycetemcomitans* and *Streptococcus sobrinus.*

As used herein, the term "oral care composition" refers to a composition that is delivered to the oral surfaces. The composition may be a product which, during the normal course of usage, is not, the purpose of systemic administration of particular therapeutic agents, intentionally swallowed, but is rather retained in the oral cavity for a time sufficient to contact substantially all of the dental surfaces and/or oral tissues for the purposes of oral activity. Examples of such compositions include, but are not limited to, toothpaste or a dentifrice, a mouthwash or a mouth rinse, a topical oral gel, a denture cleanser, and the like.

As used herein, the term "dentifrice" means paste, gel, or liquid formulations unless otherwise specified.. A dentifrice composition can be a combination of pastes, gels or paste and gel. A dentifrice composition can be a combination of pastes, gels or paste and gel.

Bacteria require certain substances in order to enable them to grow, multiply, be metabolically active and to colonize. Certain substances can be used selectively by certain microorganisms such as certain bacteria to favor their growth, metabolic activity, multiplication and colonization, and thereby directly or indirectly suppress the growth of other microorganisms.

Arginine has been found to have a greater effect promoting growth of beneficial oral bacteria relative to pathogenic bacteria, including with respect to bacterial growth, biofilm formation and competitive growth in biofilm. Arginine can be used in methods of reducing total pathogenic oral bacteria load within the oral cavity. Arginine can be used in methods of changing plaque composition towards a healthier state with less pathogens.

An assessment of impact of arginine on growth of beneficial oral bacteria and pathogenic oral bacteria demonstrated that while both beneficial oral bacteria over pathogenic oral bacteria can metabolize arginine, arginine stimulated beneficial oral bacteria to grow at an increased rate of growth while no corresponding effect was seen in pathogenic oral bacteria. In some embodiments, arginine present at a concentration of 20-25 micromol/ml preferentially promotes growth of beneficial oral bacteria over pathogenic oral bacteria. Arginine present at a concentration of 20-25 micromol/ml is particularly effective at preferentially promoting growth of *A. viscosus, S. sanguinis,* and *S. gordonii.*

An assessment of impact of arginine on biofilm formation by beneficial oral bacteria and biofilm formation by pathogenic oral bacteria demonstrated that the presence of arginine promoted total biofilm growth by *A. actinomycetemcomitans.*

Studies using dual species biofilms in which beneficial bacterial species was co-cultured with pathogenic bacteria demonstrate that the presence of arginine resulted in an increase of beneficial bacteria relative to pathogenic bacteria in biofilms. In some embodiments, arginine promotes the growth of beneficial bacteria with a concurrent reduction in the number of pathogenic bacteria such as *P. gingivalis, P. intermedia, S. mutans* and *S. sobrinus.*

Arginine as a selective nutrient has an important impact in the alteration of the oral microbiome composition by providing a competitive advantage to beneficial bacteria, helping them out-grow and out-compete pathogenic microbes within the oral cavity. The reduction of total pathogen load within the oral cavity could have important oral health implications.

Arginine may be provided in oral care compositions to improve overall oral health. Arginine may be provided in oral care compositions including, but not limited to, dentrifice and mouthrinse formulations. Oral care compositions that comprise arginine may be used to selectively promote, in an oral cavity, a) the growth of beneficial oral bacteria relative to the growth of pathogenic oral bacteria, b) biofilm formation by beneficial oral bacteria relative to biofilm formation by pathogenic oral bacteria and c) beneficial oral bacteria growth in biofilm relative to growth of pathogenic oral bacteria in biofilm. Oral care compositions that comprise arginine may be used to maintain and/or re-establish a healthy oral microbiota.

Oral care composition comprising arginine for use in a method as further defined in the claims are provided. In some embodiments, such methods selectively promote, growth of beneficial oral bacteria relative to growth of pathogenic oral bacteria in such a population of bacteria in an individual's oral cavity. The compositions for use as defined in the claims may selectively promote, growth of beneficial oral bacteria relative to growth of pathogenic oral bacteria in biofilm that comprises beneficial oral bacteria and pathogenic oral bacteria. In some embodiments, the compositions for use as defined in the claims selectively promote, growth of beneficial oral bacteria relative to growth of pathogenic oral bacteria in biofilm in an individual's oral cavity. The compositions for use as defined in the claims may maintain and/or re-establish a healthy oral microbiota. The compositions for use as defined in the claims may selectively promote, in an oral cavity, biofilm formation by beneficial oral bacteria relative to biofilm formation by pathogenic oral bacteria.

Methods may comprise the step of treating or supplementing the oral cavity of an individual by applying or contacting the oral cavity of the individual with an effective amount of arginine, preferably an oral care composition that comprises arginine, such as for example a dentifrice that comprises arginine, a toothpaste that comprises arginine, a gel that comprises arginine, a tooth powder that comprises arginine, a mouthwash that comprises arginine, a mouth rinse that comprises arginine, a lozenge which may be dissolvable or chewable and which comprises arginine, a tablet that comprises arginine, a spray that comprises arginine, a gum that comprises arginine, or a film which may be wholly or partially dissolvable, or indissolvable and which comprises arginine. In certain embodiments, the contacting of the oral cavity with the oral care composition that comprises arginine comprises the step of applying the oral care composition to the oral cavity using a brush, rinsing the oral cavity with the oral care composition in the form of a mouthwash, or spraying the oral care composition into the oral cavity using, for example, an atomizer. The individual or subject may be a mammal. In some embodiments, the individual or subject is a human. In some embodiments, the individual or subject is an animal, for example a companion animal (e.g. a cat or dog).

The oral cavity of the individual is contacted with an effective amount of arginine. An effective amount of arginine selectively promotes growth of beneficial oral bacteria relative to growth of pathogenic oral bacteria, and/or selectively promotes biofilm formation by beneficial oral bacteria relative to biofilm formation by pathogenic oral bacteria and/or selectively promotes beneficial oral bacteria growth in biofilm relative to growth of pathogenic oral bacteria in biofilm, and/or maintains and/or re-establish a healthy oral microbiota.

In some embodiments, the oral cavity of an individual is contacted with arginine in an amount sufficient to provide arginine in the oral cavity at a concentration of at least 5 micromol/ml, in some embodiments at least 10 micromol/ml, in some embodiments at least 20 micromol/ml, in some embodiments at least 25 micromol/ml or more, in some embodiments about 20-25 micromol/ml, in some embodiments about 0.05-0.1 mg/ml, in some embodiments about 0.1-1.0 mg/ml or more, in some embodiments about 1.0-5.0 mg/ml, in some embodiments about 5-10 mg/ml, in some embodiments about 10-15 mg/ml, in some embodiments about 1 mg/ml, about 2 mg/ml, about 3 mg/ml, about 4 mg/ml, about 5 mg/ml, about 6 mg/ml, about 7 mg/ml, about 8 mg/ml, about 9 mg/ml, about 10 mg/ml, about 11 mg/ml, about 12 mg/ml, about 13 mg/ml, about 14 mg/ml or about 15 mg/ml.

In some embodiments, the oral cavity of an individual is contacted with 0.1-50 mg of arginine, in some embodiments, 0.25-50 mg of arginine, in some embodiments, 0.5-50 mg of arginine, in some embodiments, 1-50 mg of arginine, in some embodiments, 5-50 mg, of arginine in some embodiments, 10-40 mg of arginine, in some embodiments, 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg of, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg, or 50 mg of arginine.

The pathogenic oral bacteria are *Porphyromonas gingivalis, Prevotella intermedia, Streptococcus mutans* and *Streptococcus sobrinus.*

In some embodiments, the beneficial oral bacteria include one or more beneficial oral bacteria species selected from the group consisting of: *Streptococcus gordonii, Actinomyces viscosus, Streptococcus salivarius, Streptococcus oralis, Streptococcus mitis* and *Streptococcus sanguinis.*

One or more beneficial oral bacteria species selected from the group consisting of: *Streptococcus gordonii, Actinomyces viscosus, Streptococcus salivarius, Streptococcus oralis, Streptococcus mitis* and *Streptococcus sanguinis* can be identified as being present in the oral cavity of the individual.

The compositions for use in methods as defined in the claims may be useful as methods of preventing one or more of gingivitis, periodontitis, peri-implantitis, peri-implant mucositis, necrotizing gingivitis, necrotizing periodontitis and caries.

In some embodiments, the methods selectively promote biofilm formation by beneficial oral bacteria, relative to biofilm formation by pathogenic oral bacteria, after 48 hours incubation with the beneficial oral bacteria and the pathogenic oral bacteria.

Oral compositions which comprise arginine are used to apply the arginine to the oral cavity. In some embodiments, the oral compositions comprise arginine and a source of zinc ions. In some embodiments, the oral compositions comprise arginine, a source of zinc ions and a source of fluoride ions. The source of zinc ions may include zinc oxide particles such as zinc oxide particles that are from 1 to 7 microns. The source of zinc ions may be selected from zinc citrate, zinc sulfate, zinc silicate, zinc lactate, zinc phosphate, zinc oxide, or a combination thereof. The source of zinc ions may be a combination of zinc oxide and zinc citrate. The source of fluoride may be stannous fluoride.

Oral care compositions comprise arginine or a salt thereof. In some embodiments, the arginine is L- arginine or a salt thereof. Suitable salts include salts known in the art to be pharmaceutically acceptable salts are generally considered to be physiologically acceptable in the amounts and concentrations provided. Physiologically acceptable salts include those derived from pharmaceutically acceptable inorganic or organic acids or bases, for example acid addition salts formed by acids which form a physiological acceptable anion, e.g., hydrochloride or bromide salt, and base addition salts formed by bases which form a physiologically acceptable cation, for example those derived from alkali metals such as potassium and sodium or alkaline earth metals such as calcium and magnesium. Physiologically acceptable salts may be obtained using standard procedures known in the art, for example, by reacting a sufficiently basic compound such as an amine with a suitable acid affording a physiologically acceptable anion. In some embodiments, the arginine in partially or wholly in salt form such as arginine phosphate, arginine hydrochloride or arginine bicarbonate. In some embodiments, the arginine is present in an amount corresponding to 0.1% to 15%, e.g., 0.1 wt % to 10 wt %, e.g., 0.1 to 5 wt%, e.g., 0.5 wt % to 3 wt % of the total composition weight, about e.g., 1%, 1.5%, 2%, 3%, 4%, 5%, or 8%, wherein the weight of the arginine is calculated as free form. In some embodiments the arginine is present in an amount corresponding to about 0.5 wt. % to about 20 wt. % of the total composition weight, about 0.5 wt. % to about 10 wt. % of the total composition weight, for example about 1.5 wt. %, about 3.75 wt. %, about 5 wt. %, or about 7.5 wt. % wherein the weight of the arginine is calculated as free form. In some embodiments, the arginine is present in an amount of from 0.5 weight % to 10 weight %, or from 0.5 weight % to 3 weight % or from 1 weight % to 2.85 weight %, or from 1.17 weight % to 2.25 weight %, based or from 1.4 weight % to 1.6 weight %, or from 0.75 weight % to 2.9 weight %, or from 1.3 weight % to 2 weight %, or about 1.5 weight %, based on the total weight of the composition. Typically, the arginine is present in an amount of up to 5% by weight, further optionally from 0.5 to 5% by weight, still further optionally from 2.5 to 4.5% by weight, based on the total weight of the oral care composition. In some embodiments, arginine is present in an amount from 0.1 wt. % - 6.0 wt. %. (e.g., about 1.5 wt %) or from about 4.5 wt. % - 8.5 wt. % (e.g., 5.0%) or from 3.5 wt. % - 9 wt. % or 8.0 wt. %. In some embodiments, the arginine is present in a dentifrice, at for example about 0.5-2 wt. %, e.g., and about 1% in the case of a mouthwash.

In some embodiments the oral care compositions comprise arginine in combination with ZnO. Such oral care compositions may optionally further comprise a fluoride source. In some embodiments, the ZnO particles may have an average particle size of from 1 to 7 microns. In some embodiments, the ZnO particles have an average particle size of 5 microns or less. In some embodiments, in addition to zinc oxide the composition may comprise other metal oxides as well such as stannous oxide, titanium oxide, calcium oxide, copper oxide and iron oxide or a mixture thereof. In some embodiments, suitable zinc oxide particles for oral care compositions have, for example, a particle size distribution of 3 to 4 microns, or alternatively, a particle size distribution of 5 to 7 microns, alternatively, a particle size distribution of 3 to 5 microns, alternatively, a particle size distribution of 2 to 5 microns, or alternatively, a particle size distribution of 2 to 4 microns. Zinc oxide may have a particle size which is a median particle size. Suitable particles may have, for example, a median particle size of 8 microns or less, alternatively, a median particle size of 3 to 4 microns, alternatively, a median particle size of 5 to 7 microns, alternatively, a median particle size of 3 to 5 microns, alternatively, a median particle size of 2 to 5 microns, or alternatively, a median particle size of 2 to 4 microns. In another aspect, that particle size is an average (mean) particle size. In an embodiment, the mean particle comprises at least 5%, at least 10%, at least 15%, at least 20%, at least 25%, at least 30%, at least 35%, or at least 40% of the total metal oxide particles in an oral care composition of the invention. The particle may be present in an amount of up to 5% by weight, based on the total weight of the oral care composition, for example in an amount of from 0.5 to 5% by weight, preferably of up to 2% by weight, more preferably from 0.5 to 2% by weight, more preferably from 1 to 2% by weight, or in some embodiment from 2.5 to 4.5% by weight, being based on the total weight of the oral care composition. In some embodiments, the source of zinc oxide particles and/or the form they may be incorporated into the oral care composition in is selected from one or more of a powder, a nanoparticle solution or suspension, or encapsulated in a polymer or bead. Zinc oxide particles may be selected to achieve occlusion of dentin particles. Particle size distribution may be measured using a Malvern Particle Size Analyzer, Model Mastersizer 2000 (or comparable model) (Malvern Instruments, Inc., Southborough, Mass.), wherein a helium- neon gas laser beam is projected through a transparent cell which contains silica, such as, for example, silica hydrogel particles suspended in an aqueous solution. Light rays which strike the particles are scattered through angles which are inversely proportional to the particle size. The photodetector arrant measures the quantity of light at several predetermined angles. Electrical signals proportional to the measured light flux values are then processed by a microcomputer system, against a scatter pattern predicted from theoretical particles as defined by the refractive indices of the sample and aqueous dispersant to determine the particle size distribution of the metal oxide. It will be understood that other methods of measuring particle size are known in the art, and based on the disclosure set forth herein, the skilled artisan will understand how to calculate median particle size, mean particle size, and/ or particle size distribution of metal oxide particles.

In some embodiments the oral care compositions comprise arginine in combination with a zinc ion source is selected from zinc oxide, zinc citrate, zinc lactate, zinc chloride, zinc acetate, zinc gluconate, zinc glycinate, zinc sulphate, sodium zinc citrate, zinc silicate, zinc phosphate, and combinations thereof. Such oral care compositions may optionally further comprise a fluoride source. Examples of effective amount of zinc ions is an amount of zinc effective inhibit erosion, e.g., from 0.005-5% zinc, e.g., 0.01 - 0.05% for a mouthwash or 0.1 to 3% for a dentifrice, e.g., a dentifrice comprising 1-3% zinc citrate.

In some embodiments the oral care compositions comprise arginine in combination with two or more zinc salts wherein at least one is zinc oxide and at least one is zinc citrate... Such oral care compositions may optionally further comprise a fluoride source. In some embodiments, the weight ratio or zinc oxide to zinc citrate is 1.5:1 to 4.5:1, 1.5:1 to 4:1, 1.7:1 to 2.3:1, 1.9:1 to 2.1:1, or about 2:1. Also, the corresponding molar ratios based on these weight ratios can be used. In some embodiments, the total concentration of zinc salts in the composition is from 0.2 weight % to 5 weight %, or from 0.5 weight % to 2.5 weight % or from 0.8 weight % to 2 weight %, or about 1.5 weight %, based on the total weight of the composition. In some embodiments, the molar ratio of arginine to total zinc salts is from 0.05:1 to 10:1. In some embodiments, the composition comprises zinc oxide in an amount of from 0.5 weight % to 1.5 weight % and zinc citrate in an amount of from 0.25 weight % to 0.75 weight %, based on the total weight of the composition. In some embodiments, the composition may comprise zinc oxide in an amount of from 0.75 weight % to 1.25 weigh % and zinc citrate in an amount of from 0.4 weight % to 0.6 weight %, based on the total weight of the composition. In some embodiments, the composition comprises zinc oxide in an amount of about 1 weight % and zinc citrate in an amount of about 0.5 weight %, based on the total weight of the composition. In some embodiments, zinc oxide may be present in an amount of from 0.75 to 1.25 wt% (e.g., 1.0 wt. %) the zinc citrate is in an amount of from 0.25 to 1.0 wt% (e.g. 0.25 to 0.75 wt. %, or 0.5 wt. %) and based on the weight of the oral care composition. In some embodiments, the zinc citrate is about 0.5 wt%. In some embodiments, the zinc oxide is about 1.0 wt%.

One or more fluoride ion sources are optionally present in an amount providing a clinically efficacious amount of soluble fluoride ion to the oral care composition. A fluoride ion source is useful, for example, as an anti-caries agent. Any orally acceptable particulated fluoride ion source can be used, including stannous fluoride, sodium fluoride, potassium fluoride, potassium monofluorophosphate, sodium monofluorophosphate, ammonium monofluorophosphate, sodium fluorosilicate, ammonium fluorosilicate, indium fluoride, amine fluoride such as olaflur (N'-octadecyltrimethylendiamine-N,N,N'-tris(2-ethanol)-dihydrofluoride), ammonium fluoride, titanium fluoride, hexafluorosulfate, and combinations thereof. Fluoride where present may be present at levels of, e.g., about 25 to about 25,000 ppm, for example about 50 to about 5000 ppm, about 750 to about 2,000 ppm for a consumer toothpaste (e.g., 1000-1500 ppm, e.g., about 1000 ppm, e.g., about 1450ppm)., product. In some embodiments, fluoride is present from about 100 to about 1000, from about 200 to about 500, or about 250 ppm fluoride ion. 500 to 3000 ppm. In some embodiments, the fluoride source provides fluoride ion in an amount of from 50 to 25,000 ppm (e.g., 750 -7000 ppm, e.g., 1000-5500 ppm, e.g., about 500 ppm, 1000 ppm, 1100 ppm, 2800 ppm, 5000 ppm, or 25000 ppm). In some embodiments, the fluoride source is stannous fluoride. In some embodiments, the fluoride source is stannous fluoride which provides fluoride in an amount from 750 - 7000 ppm (e.g., about 1000 ppm, 1100 ppm, 2800 ppm, 5000 ppm). In some embodiments, the fluoride source is stannous fluoride which provides fluoride in an amount of about 5000 ppm. In some embodiments, the fluoride source is sodium fluoride which provides fluoride in an amount from 750 - 2000ppm (e.g., about 1450ppm). In some embodiments, the fluoride source is selected from sodium fluoride and sodium monofluorophosphate and which provides fluoride in an amount from 1000ppm -1500ppm. In some embodiments, the fluoride source is sodium fluoride or sodium monofluorophosphate and which provides fluoride in an amount of about 1450ppm. In some embodiments, stannous fluoride is the only fluoride source. Fluoride ion sources may be added to the compositions at a level of about 0.001 wt. % to about 10 wt. %, e.g., from about 0.003 wt. % to about 5 wt. %, 0.01 wt. % to about 1 wt., or about 0.05 wt. %. However, it is to be understood that the weights of fluoride salts to provide the appropriate level of fluoride ion will obviously vary based on the weight of the counter ion in the salt, and one of skill in the art may readily determine such amounts. In some embodiment, the fluoride source is a fluoride salt present in an amount of 0.1 wt. % to 2 wt. % (0.1 wt% - 0.6 wt. %) of the total composition weight (e.g., sodium fluoride (e.g., about 0.32 wt. %) or sodium monofluorophosphate). e.g., 0.3-0.4%, e.g., ca. 0.32% sodium fluoride

The oral care compositions described herein may also comprise one or more further agents such as those typically selected from the group consisting of: abrasives, an anti-plaque agent, a whitening agent, antibacterial agent, cleaning agent, a flavoring agent, a sweetening agent, adhesion agents, surfactants, foam modulators, pH modifying agents, humectants, mouth-feel agents, colorants, tartar control (anti-calculus) agent, polymers, saliva stimulating agent, nutrient, viscosity modifier, anti-sensitivity agent, antioxidant, and combinations thereof.

In some embodiments, the oral care compositions comprise one or more abrasive particulates such as those useful for example as a polishing agent. Any orally acceptable abrasive can be used, but type, fineness, (particle size) and amount of abrasive should be selected so that tooth enamel is not excessively abraded in normal use of the composition. Examples of abrasive particulates may be used include abrasives such sodium bicarbonate, insoluble phosphates (such as orthophosphates, polymetaphosphates and pyrophosphates including dicalcium orthophosphate dihydrate, calcium pyrophosphate, tricalcium phosphate, calcium polymetaphosphate and insoluble sodium polymetaphosphate), calcium phosphate (e.g., dicalcium phosphate dihydrate), calcium sulfate, natural calcium carbonate (CC), precipitated calcium carbonate (PCC), silica (e.g., hydrated silica or silica gels or in the form of precipitated silica or as admixed with alumina), iron oxide, aluminum oxide, aluminum silicate, calcined alumina, bentonite, other siliceous materials, perlite, plastic particles, e.g., polyethylene, and combinations thereof. The natural calcium carbonate abrasive of is typically a finely ground limestone which may optionally be refined or partially refined to remove impurities. The material preferably has an average particle size of less than 10 microns, e.g., 3-7 microns, e.g. about 5.5 microns. For example, a small particle silica may have an average particle size (D50) of 2.5 - 4.5 microns. Because natural calcium carbonate may contain a high proportion of relatively large particles of not carefully controlled, which may unacceptably increase the abrasivity, preferably no more than 0.01%, preferably no more than 0.004%) by weight of particles would not pass through a 325 mesh. The material has strong crystal structure, and is thus much harder and more abrasive than precipitated calcium carbonate. The tap density for the natural calcium carbonate is for example between 1 and 1.5 g/cc, e.g., about 1.2 for example about 1.19 g/cc. There are different polymorphs of natural calcium carbonate, e.g., calcite, aragonite and vaterite, calcite being preferred for purposes of this invention. An example of a commercially available product suitable for use in the present invention includes Vicron ^{®} 25-11 FG from GMZ. Precipitated calcium carbonate has a different crystal structure from natural calcium carbonate. It is generally more friable and more porous, thus having lower abrasivity and higher water absorption. For use in the present invention, the particles are small, e.g., having an average particle size of 1-5 microns, and e.g., no more than 0.1 %, preferably no more than 0.05% by weight of particles which would not pass through a 325 mesh. The particles may for example have a D50 of 3-6 microns, for example 3.8-4.9, e.g., about 4.3; a D50 of 1-4 microns, e.g. 2.2-2.6 microns, e.g., about 2.4 microns, and a D10 of 1-2 microns, e.g., 1.2-1.4, e.g. about 1.3 microns. The particles have relatively high-water absorption, e.g., at least 25 g/100 g, e.g. 30-70 g/100 g. Examples of commercially available products suitable for use include, for example, Carbolag^{®} 15 Plus from Lagos Industria Quimica. In some embodiments, additional calcium-containing abrasives, for example calcium phosphate abrasive, e.g., tricalcium phosphate, hydroxyapatite or dicalcium phosphate dihydrate or calcium pyrophosphate, and/or silica abrasives, sodium metaphosphate, potassium metaphosphate, aluminum silicate, calcined alumina, bentonite or other siliceous materials, or combinations thereof are used. Examples of silica abrasives include, but are not limited to, precipitated or hydrated silicas having a mean particle size of up to about 20 microns (such as Zeodent 105 and Zeodent 114 marketed by J.M. Huber Chemicals Division, Havre de Grace, Md. 21078); Sylodent 783 (marketed by Davison Chemical Division of W.R. Grace & Company); or Sorbosil AC 43 (from PQ Corporation). In some embodiments, an effective amount of a silica abrasive is about 10-30%, e.g. about 20%. In some embodiments, the acidic silica abrasive Sylodent is included at a concentration of about 2 to about 35% by weight; about 3 to about 20 % by weight, about 3 to about 15% by weight, about 10 to about 15 % by weight. For example, the acidic silica abrasive may be present in an amount selected from 2 wt.%, 3wt.%, 4% wt.%, 5 wt.%, 6 wt.%, 7 wt.%, 8 wt.%, 9 wt.%, 10 wt.%, 11 wt.%, 12 wt.%, 13 wt.%, 14 wt.%,15 wt.%, 16 wt.%, 17 wt.%, 18 wt.%, 19 wt.%, 20 wt.%. Sylodent 783 has a pH of 3.4-4.2 when measured as a 5% by weight slurry in water and silica material has an average particle size of less than 10 microns, e.g., 3-7 microns, e.g. about 5.5 microns. In some embodiments, the silica is synthetic amorphous silica, (e.g., 1% - 28% by wt.) (e.g., 8% - 25% by wt). In some embodiments, the silica abrasives are silica gels or precipitated amorphous silicas, e.g. silicas having an average particle size ranging from 2.5 microns to 12 microns. Some embodiments further comprise a small particle silica having a median particle size (d50) of 1- 5 microns (e.g., 3-4 microns) (e.g., about 5 wt. % Sorbosil AC43 from PQ Corporation Warrington, United Kingdom). The composition may contain from 5 to 20 wt % small particle silica, or for example 10 - 15 wt %, or for example 5 wt %, 10 wt%, 15 wt % or 20 wt % small particle silica. In some embodiments, 20-30 wt% of the total silica in the composition is small particle silica (e.g., having a median particle size (d50) of 3-4 microns and wherein the small particle silica is about 5 wt. % of the oral care composition. In some embodiments, silica is used as a thickening agent, e.g., particle silica. In some embodiments, the composition comprises calcium carbonate, such as precipitated calcium carbonate high absorption (e.g., 20% to 30% by weight of the composition or, 25% precipitated calcium carbonate high absorption), or precipitated calcium carbonate - light (e.g., about 10% precipitated calcium carbonate - light) or about 10% natural calcium carbonate.

In some embodiments, the oral care compositions comprise a whitening agent, e.g., a selected from the group consisting of peroxides, metal chlorites, perborates, percarbonates, peroxyacids, hypochlorites, hydroxyapatite, and combinations thereof. Oral care compositions may comprise hydrogen peroxide or a hydrogen peroxide source, e.g., urea peroxide or a peroxide salt or complex (e.g., such as peroxyphosphate, peroxycarbonate, perborate, peroxysilicate, or persulphate salts; for example, calcium peroxyphosphate, sodium perborate, sodium carbonate peroxide, sodium peroxyphosphate, and potassium persulfate or hydrogen peroxide polymer complexes such as hydrogen peroxide-polyvinyl pyrrolidone polymer complexes.

In some embodiments, the oral care compositions comprise an effective amount of one or more antibacterial agents, for example comprising an antibacterial agent selected from halogenated diphenyl ether (e.g. triclosan), triclosan monophosphate, herbal extracts and essential oils (e.g., rosemary extract, tea extract, magnolia extract, thymol, menthol, eucalyptol, geraniol, carvacrol, citral, hinokitol, magonol, ursolic acid, ursic acid, morin, catechol, methyl salicylate, epigallocatechin gallate, epigallocatechin, gallic acid, miswak extract, sea-buckthorn extract), bisguanide antiseptics (e.g., chlorhexidine, alexidine or octenidine), quaternary ammonium compounds (e.g., cetylpyridinium chloride (CPC), benzalkonium chloride, tetradecylpyridinium chloride (TPC), N-tetradecyl-4-ethylpyridinium chloride (TDEPC)), phenolic antiseptics, hexetidine furanones, bacteriocins, ethyllauroyl arginate, arginine bicarbonate, a Camellia extract, a flavonoid, a flavan, halogenated diphenyl ether, creatine, sanguinarine, povidone iodine, delmopinol, salifluor, metal ions (e.g., zinc salts, stannous salts, copper salts, iron salts), propolis and oxygenating agents (e.g., hydrogen peroxide, buffered sodium peroxyborate or peroxycarbonate), phthalic acid and its salts, monoperthalic acid and its salts and esters, ascorbyl stearate, oleoyl sarcosine, alkyl sulfate, dioctyl sulfosuccinate, salicylanilide, domiphen bromide, delmopinol, octapinol and other piperidino derivatives, nisin preparations, chlorite salts; parabens such as methylparaben or propylparaben and mixtures of any of the foregoing. One or more additional antibacterial or preservative agents may optionally be present in the composition in a total amount of from about 0.01 wt. % to about 0.5 wt. %, optionally about 0.05 wt. % to about 0.1 wt. % or about 0.3%. by total weight of the composition.

In some embodiments, the oral care compositions may comprise at least one bicarbonate salt useful for example to impart a "clean feel" to teeth and gums due to effervescence and release of carbon dioxide. Any orally acceptable bicarbonate can be used, including without limitation, alkali metal bicarbonates such as sodium and potassium bicarbonates, ammonium bicarbonate and the like. The one or more additional bicarbonate salts are optionally present in a total amount of about 0.1 wt. % to about 50 wt. %, for example about 1 wt. % to 20 wt. %, by total weight of the composition.

In some embodiments, the oral care compositions also comprise at least one flavorant, useful for example to enhance taste of the composition. Any orally acceptable natural or synthetic flavorant can be used, including without limitation essential oils and various flavoring aldehydes, esters, alcohols, and similar materials, tea flavors, vanillin, sage, marjoram, parsley oil, spearmint oil, cinnamon oil, oil of wintergreen, peppermint oil, clove oil, bay oil, anise oil, eucalyptus oil, citrus oils, fruit oils, sassafras and essences including those derived from lemon, orange, lime, grapefruit, apricot, banana, grape, apple, strawberry, cherry, pineapple, etc., bean- and nut- derived flavors such as coffee, cocoa, cola, peanut, almond, etc., adsorbed and encapsulated flavorants and the like. Also encompassed within flavorants herein are ingredients that provide fragrance and/or other sensory effect in the mouth, including cooling or wanning effects. Such ingredients illustratively include menthol, carvone, methyl acetate, menthyl lactate, camphor, eucalyptus oil, eucalyptol, anethole, eugenol, cassia, oxanone, a-irisone, propenyl guaiethoi, thymol, linalool, benzaldehyde, cinnamaldehyde, N-ethyl-p-menthan-3-carboxamine, N,2,3-trimethyl-2- isopropylbutanamide, 3-(1-menthoxy)-propane-1,2-diol, cinnamaldehyde glycerol acetal (CGA), menthone glycerol acetal (MGA) and the like. One or more flavorants are optionally present in a total amount of from about 0.01 wt. % to about 5 wt. %, for example, from about 0.03 wt. % to about 2.5 wt.%, optionally about 0.05 wt.% to about 1.5 wt.%, further optionally about 0.1 wt.% to about 0.3 wt.% and in some embodiments in various embodiments from about 0.01 wt. % to about 1 wt. %, from about 0.05 to about 2%, from about 0.1% to about 2.5%, and from about 0.1 to about 0.5% by total weight of the composition.

In some embodiments, the oral care compositions comprise at least one sweetener, useful for example to enhance taste of the composition. Sweetening agents among those useful herein include dextrose, polydextrose, sucrose, maltose, dextrin, dried invert sugar, mannose, xylose, ribose, fructose, levulose, galactose, corn syrup, partially hydrolyzed starch, hydrogenated starch hydrolysate, ethanol, sorbitol, mannitol, xylitol, maltitol, isomalt, aspartame, neotame, saccharin and salts thereof (e.g. sodium saccharin), sucralose, dipeptide-based intense sweeteners, cyclamates, dihydrochalcones, glycerine, propylene glycol, polyethylene glycols, Poloxomer polymers such as POLOXOMER 407, PLURONIC F108, (both available from BASF Corporation), alkyl polyglycoside (APG), polysorbate, PEG40, castor oil, menthol, and mixtures thereof. One or more sweeteners are optionally present in a total amount depending strongly on the particular sweetener(s) selected, but typically 0.005 wt.% to 5 wt.%, by total weight of the composition, optionally 0.005 wt.% to 0.2 wt.%, further optionally 0.05 wt.% to 0.1 wt.% by total weight of the composition.

In some embodiments, the oral care compositions further comprises an agent that interferes with or prevents bacterial attachment, e.g., ethyl lauroyl arginiate (ELA), solbrol or chitosan, as well as plaque dispersing agents such as enzymes (papain, glucoamylase, etc.).

In some embodiments, the oral care compositions also comprise at least one surfactant. Any orally acceptable surfactant, most of which are anionic, cationic, zwitterionic, nonionic or amphoteric, and mixtures thereof, can be used. Examples of suitable surfactants include water-soluble salts of higher fatty acid monoglyceride monosulfates, such as the sodium salt of monosulfated monoglyceride of hydrogenated coconut oil fatty acids; higher alkyl sulfates such as sodium lauryl sulfate, sodium coconut monoglyceride sulfonate, sodium lauryl sarcosinate, sodium lauryl isoethionate, sodium laureth carboxylate and sodium dodecyl benzenesulfonate; alkyl aryl sulfonates such as sodium dodecyl benzene sulfonate; higher alkyl sulfoacetates, such as sodium lauryl sulfoacetate; higher fatty acid esters of 1,2-dihydroxypropane sulfonate; and the substantially saturated higher aliphatic acyl amides of lower aliphatic amino carboxylic compounds, such as those having 12-16 carbons in the fatty acid, alkyl or acyl radicals; and the like. Examples of amides include N-lauryl sarcosine, and the sodium, potassium and ethanolamine salts of N-lauryl, N-myristoyl, or N-palmitoyl sarcosine. Examples of cationic surfactants include derivatives of aliphatic quaternary ammonium compounds having one long alkyl chain containing 8 to 18 carbon atoms such as lauryl trimethylammonium chloride, cetyl pyridinium chloride, cetyl trimethyl ammonium bromide, di-isobutylphenoxyethyldimethylbenzylammonium chloride, coconut alkyltrimethylammonium nitrite, cetyl pyridinium fluoride, and mixtures thereof. Suitable nonionic surfactants include without limitation, poloxamers, polyoxyethylene sorbitan esters, fatty alcohol ethoxylates, alkylphenol ethoxylates, tertiary amine oxides, tertiary phosphine oxides, di alkyl sulfoxides and the like. Others include, for example, non-anionic polyoxyethylene surfactants, such as Polyoxamer 407, Steareth 30, Polysorbate 20, and castor oil; and amphoteric surfactants such as derivatives of aliphatic secondary and tertiary amines having an anionic group such as carboxylate, sulfate, sulfonate, phosphate or phosphonate such as cocamidopropyl betaine (tegobaine), and cocamidopropyl betaine lauryl glucoside; condensation products of ethylene oxide with various hydrogen containing compounds that are reactive therewith and have long hydrocarbon chains (e.g., aliphatic chains of from 12 to 20 carbon atoms), which condensation products (ethoxamers) contain hydrophilic polyoxyethylene moieties, such as condensation products of poly (ethylene oxide) with fatty acids, fatty, alcohols, fatty amides and other fatty moieties, and with propylene oxide and polypropylene oxides. In some embodiments, the oral composition includes a surfactant system that is sodium laurel sulfate (SLS) and cocamidopropyl betaine. One or more surfactants are optionally present in a total amount of about 0.01 wt.% to about 10 wt. %, for example, from about 0.05 wt. % to about 5 wt. %, or from about 0.1 wt. % to about 2 wt. %, e.g 1.5% wt. by total weight of the composition. In some embodiments, the oral composition include an anionic surfactant, e.g., a surfactant selected from sodium lauryl sulfate, sodium ether lauryl sulfate, and mixtures thereof, e.g. in an amount of from about 0.3% to about 4.5% by weight, e.g. 1-2% sodium lauryl sulfate (SLS); and/or a zwitterionic surfactant, for example a betaine surfactant, for example cocamidopropylbetaine, e.g. in an amount of from about 0.1% to about 4.5% by weight, e.g. 0.5-2% cocamidopropylbetaine. Some embodiments comprise a nonionic surfactant in an amount of from 0.5 -5%, e.g, 1-2%, selected from poloxamers (e.g., poloxamer 407), polysorbates (e.g., polysorbate 20), polyoxyl hydrogenated castor oil (e.g., polyoxyl 40 hydrogenated castor oil), and mixtures thereof. In some embodiments, the poloxamer nonionic surfactant has a polyoxypropylene molecular mass of from 3000 to 5000 g/mol and a polyoxyethylene content of from 60 to 80 mol%, e.g., the poloxamer nonionic surfactant comprises poloxamer 407. Any of the preceding compositions may further comprise sorbitol, wherein the sorbitol is in a total amount of 10- 40% (e.g., about 23%).

In some embodiments, the oral care compositions comprise at least, one foam modulator, useful for example to increase amount, thickness or stability of foam generated by the composition upon agitation. Any orally acceptable foam modulator can be used, including without limitation, polyethylene glycols (PEGs), also known as polyoxyethylenes. High molecular weight PEGs are suitable, including those having an average molecular weight of 200,000 to 7,000,000, for example 500,000 to 5,000,000, or 1,000,000 to 2,500,000, One or more PEGs are optionally present in a total amount of about 0.1 wt. % to about 10 wt. %, for example from about 0.2 wt. % to about 5 wt. %, or from about 0.25 wt. % to about 2 wt. %, by total weight of the composition

In some embodiments, the oral care compositions comprise at least one pH modifying agent. Such agents include acidifying agents to lower pH, basifying agents to raise pH, and buffering agents to control pH within a desired range. For example, one or more compounds selected from acidifying, basifying and buffering agents can be included to provide a pH of 2 to 10, or in various illustrative embodiments, 2 to 8, 3 to 9, 4 to 8, 5 to 7, 6 to 10, 7 to 9, etc. Any orally acceptable pH modifying agent can be used, including without limitation, carboxylic, phosphoric and sulfonic acids, acid salts (e.g., monosodium citrate, disodium citrate, monosodium malate, etc.), alkali metal hydroxides such as sodium hydroxide, carbonates such as sodium carbonate, bicarbonates such as sodium bicarbonate, sesquicarbonates, borates, silicates, bisulfates, phosphates (e.g., monosodium phosphate, trisodium phosphate, monopotassium phosphate, dipotassium phosphate, tribasic sodium phosphate, sodium tripolyphosphate, phosphoric acid), imidazole, sodium phosphate buffer (e.g., sodium phosphate monobasic and disodium phosphate) citrates (e.g. citric acid, trisodium citrate dehydrate), pyrophosphates (sodium and potassium salts) and the like and combinations thereof. One or more pH modifying agents are optionally present in a total amount effective to maintain the composition in an orally acceptable pH range. Compositions may have a pH that is either acidic or basic, e.g., from pH 4 to pH 5.5 or from pH 8 to pH 10. In some embodiments, the amount of buffering agent is sufficient to provide a pH of about 5 to about 9, preferable about 6 to about 8, and more preferable about 7, when the composition is dissolved in water, a mouthrinse base, or a toothpaste base. Typical amounts of buffering agent are about 5% to about 35%, in one embodiment about 10% to about 30%), in another embodiment about 15% to about 25%, by weight of the total composition.

In some embodiments, the oral care compositions also comprise at least one humectant. Any orally acceptable humectant can be used, including without limitation, polyhydric alcohols such as glycerin, sorbitol (optionally as a 70 wt. % solution in water), propylene glycol, xylitol or low molecular weight polyethylene glycols (PEGs) and mixtures thereof. Most humectants also function as sweeteners. In some embodiments, compositions comprise 15% to 70% or 30% to 65% by weight humectant. Suitable humectants include edible polyhydric alcohols such as glycerine, sorbitol, xylitol, propylene glycol as well as other polyols and mixtures of these humectants. Mixtures of glycerine and sorbitol may be used in certain embodiments as the humectant component of the compositions herein. One or more humectants are optionally present in a total amount of from about 1 wt.% to about 70 wt.%, for example, from about 1 wt.% to about 50 wt.%, from about 2 wt.% to about 25 wt.%, or from about 5 wt.% to about 15 wt.%, by total weight of the composition. In some embodiments, humectants, such as glycerin are present in an amount that is at least 20%>, e.g., 20-40%, e.g., 25-35%.

Mouth-feel agents include materials imparting a desirable texture or other feeling during use of the composition. In some embodiments, the oral care compositions comprise at least one thickening agent, useful for example to impart a desired consistency and/or mouth feel to the composition. Any orally acceptable thickening agent can be used, including without limitation, carbomers, also known as carboxyvinyl polymers, carrageenans, also known as Irish moss and more particularly i-carrageenan (iota-carrageenan), cellulosic polymers such as hydroxyethyl cellulose, and water-soluble salts of cellulose ethers (e.g., sodium carboxymethyl cellulose and sodium carboxymethyl hydroxyethyl cellulose), carboxymethylcellulose (CMC) and salts thereof, e.g., CMC sodium, natural gums such as karaya, xanthan, gum arabic and tragacanthin, colloidal magnesium aluminum silicate, colloidal silica, starch, polyvinyl pyrrolidone, hydroxyethyl propyl cellulose, hydroxybutyl methyl cellulose, hydroxypropyl methyl cellulose, and hydroxyethyl cellulose and amorphous silicas, and the like. A preferred class of thickening or gelling agents includes a class of homopolymers of acrylic acid crosslinked with an alkyl ether of pentaerythritol or an alkyl ether of sucrose, or carbomers. Carbomers are commercially available from B. F. Goodrich as the Carbopol^{©} series. Particularly preferred Carbopols include Carbopol 934, 940, 941, 956, 974P, and mixtures thereof. Silica thickeners such as DT 267 (from PPG Industries) may also be used. One or more thickening agents are optionally present in a total amount of from about 0.01 wt. % to 15 wt.%, for example from about 0.1 wt.% to about 10 wt.%, or from about 0.2 wt. % to about 5 wt.%, by total weight of the composition. Some embodiments comprise sodium carboxymethyl cellulose (e.g., from 0.5 wt. % - 1.5 wt. %). In certain embodiments, thickening agents in an amount of about 0.5% to about 5.0% by weight of the total composition are used. Thickeners may be present in an amount of from 1 wt % to 15 wt %, from 3 wt % to 10 wt %, 4 wt % to 9 wt %, from 5 wt % to 8 wt %, for example 5 wt %, 6 wt %, 7 wt %, or 8 wt %.

In some embodiments, the oral care compositions comprise at least one colorant. Colorants herein include pigments, dyes, lakes and agents imparting a particular luster or reflectivity such as pearling agents. In various embodiments, colorants are operable to provide a white or light-colored coating on a dental surface, to act as an indicator of locations on a dental surface that have been effectively contacted by the composition, and/ or to modify appearance, in particular color and/ or opacity, of the composition to enhance attractiveness to the consumer. Any orally acceptable colorant can be used, including FD&C dyes and pigments, talc, mica, magnesium carbonate, calcium carbonate, magnesium silicate, magnesium aluminum silicate, silica, titanium dioxide, zinc oxide, red, yellow, brown and black iron oxides, ferric ammonium ferrocyanide, manganese violet, ultramarine, titaniated mica, bismuth oxychloride, and mixtures thereof. One or more colorants are optionally present in a total amount of about 0.001% to about 20%, for example about 0.01% to about 10% or about 0.1% to about 5% by total weight of the composition.

In some embodiments, the oral care composition further comprises an anti-calculus (tartar control) agent. Suitable anti-calculus agents include, but are not limited to: phosphates and polyphosphates, polyaminopropane sulfonic acid (AM PS), polyolefin sulfonates, polyolefin phosphates, diphosphonates such as azacycloalkane-2,2-diphosphonates (e.g., azacycloheptane-2,2-diphosphonic acid), N-methyl azacyclopentane-2,3-diphosphonic acid, ethane-1-hydroxy-1,1-diphosphonic acid (EHDP) and ethane-1-amino-1,1-diphosphonate, phosphonoalkane carboxylic acids and. Useful inorganic phosphate and polyphosphate salts include monobasic, dibasic and tribasic sodium phosphates. Soluble pyrophosphates are useful anticalculus agents. The pyrophosphate salts can be any of the alkali metal pyrophosphate salts. In certain embodiments, salts include tetra alkali metal pyrophosphate, dialkali metal diacid pyrophosphate, trialkali metal monoacid pyrophosphate and mixtures thereof, wherein the alkali metals are sodium or potassium. The pyrophosphates also contribute to preservation of the compositions by lowering water activity, tetrasodium pyrophosphate (TSPP), tetrapotassium pyrophosphate, sodium tripolyphosphate, tetrapolyphosphate, sodium trimetaphosphate, sodium hexametaphosphate and mixtures thereof. The salts are useful in both their hydrated and unhydrated forms. An effective amount of pyrophosphate salt useful in the present composition is generally enough to provide least 0.1 wt. % pyrophosphate ions, e.g., 0.1 to 3 wt. %, e.g., 0.1 to 2 wt. %, e.g., 0.1 to 1 wt. %, e.g., 0.2 to 0.5 wt. %.

Other useful tartar control agents include polymers and co-polymers. In some embodiments, the oral care compositions include one or more polymers, such as polyethylene glycols, polyvinyl methyl ether maleic acid copolymers, polysaccharides (e.g., cellulose derivatives, for example carboxymethyl cellulose, or polysaccharide gums, for example xanthan gum or carrageenan gum). Acidic polymers, for example polyacrylate gels, may be provided in the form of their free acids or partially or fully neutralized water-soluble alkali metal (e.g., potassium and sodium) or ammonium salts. Certain embodiments include 1:4 to 4:1 copolymers of maleic anhydride or acid with another polymerizable ethylenically unsaturated monomer, for example, methyl vinyl ether (methoxyethylene), having a molecular weight (M.W.) of about 30,000 to about 1,000,000, polyvinyl methyl ether/maleic anhydride (PVM/MA) copolymers such as GANTREZ^{®} (e.g., GANTREZ^{®} S-97 polymer). In some embodiments, the PVM/MA copolymer comprises a copolymer of methyl vinyl ether/maleic anhydride, wherein the anhydride is hydrolyzed following copolymerization to provide the corresponding acid. In some embodiments, PVM/MA copolymer has an average molecular weight (M.W.) of about 30,000 to about 1,000,000, e.g. about 300,000 to about 800,000, e.g., wherein the anionic polymer is about 1-5%, e.g., about 2%, of the weight of the composition. In some embodiments, the anti-calculus agent is present in the composition in an amount of from 0.2 weight % to 0.8 weight %; 0.3 weight % to 0.7 weight %; 0.4 weight % to 0.6 weight %; or about 0.5 weight %, based on the total weight of the composition. Copolymers are available for example as Gantrez AN 139(M.W. 500,000), AN 1 19 (M.W. 250,000) and S-97 Pharmaceutical Grade (M.W. 70,000), of GAF Chemicals Corporation. Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1 103, M.W. 10,000 and EMA Grade 61, and 1:1 copolymers of acrylic acid with methyl or hydroxyethyl methacrylate, methyl or ethyl acrylate, isobutyl vinyl ether or N-vinyl-2-pyrrolidone. Suitable generally, are polymerized olefinically or ethyl enically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha- chlorsorbic, cinnamic, beta-styrylacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility. A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2 methylpropane sulfonic acid having a molecular weight of about 1,000 to about 2,000,000. Another useful class of polymeric agents includes polyamino acids, particularly those containing proportions of anionic surface-active amino acids such as aspartic acid, glutamic acid and phosphoserine.

In some embodiments, the oral care compositions comprise a saliva stimulating agent useful, for example, in amelioration of dry mouth. Any orally acceptable saliva stimulating agent can be used, including without limitation food acids such as citric, lactic, malic, succinic, ascorbic, adipic, fumaric and tartaric acids, and mixtures thereof. One or more saliva stimulating agents are optionally present in saliva stimulating effective total amount.

In some embodiments, the oral care compositions comprise a nutrient. Suitable nutrients include vitamins, minerals, amino acids, and mixtures thereof. Vitamins include Vitamins C and D, miamine, riboflavin, calcium pantothenate, niacin, folic acid, nicotinamide, pyridoxine, cyanocobalamin, para-aminobenzoic acid, bioflavonoids, and mixtures thereof. Nutritional supplements include amino acids (such as L-tryptophane, L-lysine, methionine, threonine, levocarnitine and L-carnitine), lipotropics (such as choline, inositol, betaine, and linoleic acid), and mixtures thereof.

In some embodiments, the oral care compositions comprise at least one viscosity modifier, useful for example to help inhibit settling or separation of ingredients or to promote redispersibility upon agitation of a liquid composition. Any orally acceptable viscosity modifier can be used, including without limitation, mineral oil, petrolatum, clays and organo-modified clays, silicas and the like. One or more viscosity modifiers are optionally present in a total amount of from about 0.01 wt. % to about 10 wt. %, for example, from about 0.1 wt.% to about 5 wt.%, by total weight of the composition.

In some embodiments, the oral care compositions comprise antisensitivity agents, e.g., potassium salts such as potassium nitrate, potassium bicarbonate, potassium chloride, potassium citrate, and potassium oxalate; capsaicin; eugenol; strontium salts; chloride salts and combinations thereof. Such agents may be added in effective amounts, e.g., from about 1 wt. % to about 20 wt. % by weight based on the total weight of the composition, depending on the agent chosen.

In some embodiments, the oral care compositions comprise an antioxidant. Any orally acceptable antioxidant can be used, including butylated hydroxy ani sole (BHA), butylated hydroxytoluene (BHT), vitamin A, carotenoids, co-enzyme Q10, PQQ, Vitamin A, Vitamin C, vitamin E, anethole-dithiothione, flavonoids, polyphenols, ascorbic acid, herbal antioxidants, chlorophyll, melatonin, and mixtures thereof.

In some embodiments, the oral care compositions comprise of one or more alkali phosphate salts, e.g., sodium, potassium or calcium salts, e.g., selected from alkali dibasic phosphate and alkali pyrophosphate salts, e.g., alkali phosphate salts selected from sodium phosphate dibasic, potassium phosphate dibasic, dicalcium phosphate dihydrate, calcium pyrophosphate, tetrasodium pyrophosphate, tetrapotassium pyrophosphate, sodium tripolyphosphate, disodium hydrogenorthophoshpate, monosodium phosphate, pentapotassium triphosphate and mixtures of any of two or more of these, e.g., in an amount of 0.01-20%, e.g., 0.1-8%, e.g., e.g., 0.1 to 5%, e.g., 0.3 to 2%, e.g., 0.3 to 1%, e.g about 0.01%, about 0.1%, about 0.5%, about 1%, about 2%, about 5%, about 6%, by weight of the composition. In some embodiments, compositions comprise tetrapotassium pyrophosphate, disodium hydrogenorthophoshpate, monosodium phosphate, and pentapotassium triphosphate. In some embodiments, compositions comprise tetrasodium pyrophosphate from 0.1 - 1.0 wt% (e.g., about .5 wt %).

In some embodiments, the oral care compositions comprise a source of calcium and phosphate selected from (i) calcium-glass complexes, e.g., calcium sodium phosphosilicates, and (ii) calcium-protein complexes, e.g., casein phosphopeptide-amorphous calcium phosphate. Any of the preceding compositions further comprising a soluble calcium salt, e.g., selected from calcium sulfate, calcium chloride, calcium nitrate, calcium acetate, calcium lactate, and combinations thereof.

In some embodiments, the oral care compositions comprise an additional ingredient selected from: benzyl alcohol, Methylisothizolinone ("MIT"), Sodium bicarbonate, sodium methyl cocoyl taurate (tauranol), lauryl alcohol, and polyphosphate. Some embodiments comprise benzyl alcohol that is present from 0.1 - 0.8 wt %., or 0.2 to 0.7 wt %, or from 0.3 to 0.6 wt %, or from 0.4 to 0.5 wt %, e.g. about 0.1 wt. %, about 0.2 wt. %, about 0.3 wt %, about 0.4 wt %, about 0.5 wt %, about 0.6 wt%, about 0.7 wt % or about 0.8 wt %.

In some embodiments, the oral care compositions comprise from 5% - 40%, e.g., 10% - 35%, e.g., about 15%, 25%, 30%, and 35% or more of water.

Methods of determining the impact of a compound or composition in combination with arginine on growth of beneficial oral bacteria and growth on pathogenic oral bacteria can be used. The methods can be used to identify a compound or composition that enhances the selective promotion of growth of beneficial oral bacteria relative to growth of pathogenic oral bacteria by arginine. The methods can comprise performing one or more beneficial oral bacteria growth test assays, one or more pathogenic oral bacteria growth test assays, one or more beneficial oral bacteria growth control assays and one or more beneficial oral bacteria growth control assays. A beneficial oral bacteria growth test assay comprises culturing a species of beneficial oral bacteria in media that comprises arginine and a test compound or composition and measuring the growth of the beneficial oral bacteria in the beneficial oral bacteria growth test assay. A pathogenic oral bacteria growth test assay comprises culturing a species of pathogenic oral bacteria in media that comprises arginine and the test compound or composition and measuring the growth of the pathogenic oral bacteria in the pathogenic oral bacteria growth test assays. A beneficial oral bacteria growth control assay comprises culturing a species of beneficial oral bacteria in media that comprises arginine free of the test compound or composition and measuring the growth of the beneficial oral bacteria in the beneficial oral bacteria growth control assay. A pathogenic oral bacteria growth control assay comprises culturing a species of pathogenic oral bacteria in media that comprises arginine free the test compound or composition and measuring the growth of the pathogenic oral bacteria in the pathogenic oral bacteria growth control assay. The ratio of the growth of the beneficial oral bacteria in the beneficial oral bacteria growth test assay to the growth of the beneficial oral bacteria in the beneficial oral bacteria growth control assay (the beneficial ratio) is calculated and the ratio of the growth of the pathogenic oral bacteria in the pathogenic oral bacteria growth test assay to the growth of the pathogenic oral bacteria in the pathogenic oral bacteria growth control assay (the pathogenic ratio) is also calculated. The beneficial ratio is then compared to the pathogenic ratio and a higher beneficial ratio compared to the pathogenic ratio indicates that the test compound or composition enhances the selective promotion of growth of beneficial oral bacteria relative to growth of pathogenic oral bacteria by arginine. Alternatively, the ratio of the growth of the beneficial oral bacteria in the beneficial oral bacteria growth test assay to the growth of the pathogenic oral bacteria in the pathogenic oral bacteria growth test assay (the test ratio) is calculated and the ratio of the growth of the beneficial oral bacteria in the beneficial oral bacteria growth control assay to the growth of the pathogenic oral bacteria in the pathogenic oral bacteria growth control assay (the control ratio) is calculated. The test ratio is then compared to the control ratio and a higher test ratio compared to the control ratio indicates that the test compound or composition enhances the selective promotion of growth of beneficial oral bacteria relative to growth of pathogenic oral bacteria by arginine.

Test assays (beneficial oral bacteria growth test assays and pathogenic oral bacteria growth test assays) can include a series of such test assays using the media supplemented with 5, 10, 20, and 25 micromol/ml of arginine. Test assays (beneficial oral bacteria growth test assays and pathogenic oral bacteria growth test assays) can use e media supplemented with at least 5 micromol/ml, at least 10 micromol/ml, at least 20 micromol/ml, at least 25 micromol/ml , about 0.05-0.1 mg/ml, about 0.1-1.0 mg/ml, about 1 mg/ml, about 2 mg/ml, about 3 mg/ml, about 4 mg/ml, about 5 mg/ml, about 6 mg/ml, about 7 mg/ml, about 8 mg/ml, about 9 mg/ml, about 10 mg/ml, about 11 mg/ml, about 12 mg/ml, about 13 mg/ml, about 14 mg/ml and/or about 15 mg/ml or arginine. Such test assays can include a series of such test assays using the media supplemented with a test compound or composition present in different concentrations. The test assays can be performed using a 96-well polystyrene microtiter plate. The bacterial cultures are incubated under conditions to which promote growth. The bacterial cultures can be incubated under anaerobic conditions or an environment containing 5% carbon dioxide. Bacterial growth can be monitored at one or more time points, such as for example daily for 1-4 days (i.e. at 24, 48, 72 and 96 hours). Growth may be measured using various known methods such as qPCR or optical density at a wavelength of 630 nm. Control assays (beneficial oral bacteria growth control assays and pathogenic oral bacteria growth control assays) can be included. Such control assays are assays that are the same as the corresponding test assays performed except in control assays no test compound or composition is included. The growth results of bacteria in the test assays are compared to the growth results of bacteria in the control assays. Rather than performing control assays, the growth results of bacteria in the test assay can be compared to reference results of known growth rates for control assays. Beneficial oral bacteria used may be selected from *Streptococcus gordonii, Actinomyces viscosus, Streptococcus salivarius, Streptococcus oralis, Streptococcus mitis* and *Streptococcus sanguinis.* Pathogenic oral bacteria used may be selected from *P Aggregatibacter actinomycetemcomitans, Fusobacterium nucleatum, Porphyromonas gingivalis, Prevotella intermedia, Streptococcus mutans* and *Streptococcus sobrinus.* The methods are useful to identify compounds and compositions which may be used in combination with arginine to reduce pathogenic load in the oral cavity and change plaque composition towards a healthier state with less pathogenic bacteria, thereby producing less damaging plaque. The methods are useful to identify compounds and compositions which may be used in combination with arginine to prevent one or more of gingivitis, periodontitis, peri-implantitis, peri-implant mucositis, necrotizing gingivitis, necrotizing periodontitis and caries.

Methods of determining the impact of a compound or composition in combination with arginine on selective growth of beneficial oral bacteria relative to growth of pathogenic oral bacteria in biofilm can be used. The methods can be used to identify a compound or composition that enhances the selective promotion of growth of beneficial oral bacteria relative to growth of pathogenic oral bacteria in biofilm by arginine. The methods comprise performing a dual species biofilm test assay, performing a dual species biofilm control assay and comparing the dual species test biofilm's beneficial oral bacteria-to-pathogenic oral bacteria load to the dual species control biofilm's beneficial oral bacteria-to-pathogenic oral bacteria load. The dual species biofilm test assay comprises co-culturing a species of beneficial oral bacteria and a species of pathogenic oral bacteria in a dual species test biofilm that comprises arginine and a test compound or composition, quantifying beneficial oral bacteria and pathogenic oral bacteria in the dual species test biofilm; and comparing the quantity of beneficial oral bacteria to the quantity of pathogenic oral bacteria in the dual species test biofilm to determine the dual species test biofilm's beneficial oral bacteria-to-pathogenic oral bacteria load. The dual species biofilm control assay comprises co-culturing a species of beneficial oral bacteria and a species of pathogenic oral bacteria in a dual species control biofilm that comprises arginine free of the test compound or composition, quantifying beneficial oral bacteria and pathogenic oral bacteria in the dual species control biofilm; and comparing the quantity of beneficial oral bacteria to the quantity of pathogenic oral bacteria in the dual species control biofilm to determine the dual species control biofilm's beneficial oral bacteria-to-pathogenic oral bacteria load. The dual species test biofilm's beneficial oral bacteria-to-pathogenic oral bacteria load is then compared to the dual species control biofilm's beneficial oral bacteria-to-pathogenic oral bacteria load. A higher beneficial oral bacteria-to-pathogenic oral bacteria load in the dual species test biofilm compared to the beneficial oral bacteria-to-pathogenic oral bacteria load in the dual species control biofilm indicates that the test compound or composition enhances the selective promotion of growth of beneficial oral bacteria relative to growth of pathogenic oral bacteria in biofilm by arginine.

Dual species biofilm test assay can include a series of such test assays using the media supplemented with 5, 10, 20, and 25 micromol/ml of arginine. Dual species biofilm test assays can use media supplemented with at least 5 micromol/ml, at least 10 micromol/ml, at least 20 micromol/ml, at least 25 micromol/ml , about 0.05-0.1 mg/ml, about 0.1-1.0 mg/ml, about 1 mg/ml, about 2 mg/ml, about 3 mg/ml, about 4 mg/ml, about 5 mg/ml, about 6 mg/ml, about 7 mg/ml, about 8 mg/ml, about 9 mg/ml, about 10 mg/ml, about 11 mg/ml, about 12 mg/ml, about 13 mg/ml, about 14 mg/ml and/or about 15 mg/ml or arginine. Such test assays can include a series of such test assays using the media supplemented with a test compound or composition present in different concentrations. The bacterial cultures are incubated under conditions to which promote growth. The bacterial cultures can be incubated under anaerobic conditions or an environment containing 5% carbon dioxide. The quantity of beneficial oral bacteria and pathogenic oral bacteria in the dual species test biofilm and the quantity of beneficial oral bacteria and pathogenic oral bacteria in the dual species control biofilm can be determined at one or more time points, such as for example daily for 1-4 days (i.e. at 24, 48, 72 and 96 hours). The quantity of beneficial oral bacteria and pathogenic oral bacteria in the dual species test biofilm and the quantity of beneficial oral bacteria and pathogenic oral bacteria in the dual species control biofilm can be determined by qPCR. Rather than performing dual species control biofilm assays, the growth results of bacteria in the dual species test biofilm assay can be compared to reference results of known growth rates for control assays. Beneficial oral bacteria used may be selected from *Streptococcus gordonii, Actinomyces viscosus, Streptococcus salivarius, Streptococcus oralis, Streptococcus mitis* and *Streptococcus sanguinis.* Pathogenic oral bacteria used may be selected from *P Aggregatibacter actinomycetemcomitans, Fusobacterium nucleatum, Porphyromonas gingivalis, Prevotella intermedia, Streptococcus mutans* and *Streptococcus sobrinus.* The methods are useful to identify compounds and compositions which may be used with arginine to reduce pathogenic load in the oral cavity and change plaque composition towards a healthier state with less pathogenic bacteria, thereby producing less damaging plaque. The methods are useful to identify compounds and compositions which may be used in combination with arginine to prevent one or more of gingivitis, periodontitis, peri-implantitis, peri-implant mucositis, necrotizing gingivitis, necrotizing periodontitis and caries.

Other methods comprise determining the impact of a compound or composition in combination with arginine on growth of biofilm produced by beneficial oral bacteria and growth of biofilm produced by pathogenic oral bacteria. The methods comprise performing one or more beneficial oral bacteria biofilm growth test assays, one or more pathogenic oral bacteria biofilm growth test assays, one or more beneficial oral bacteria biofilm growth control assays and one or more beneficial oral bacteria biofilm growth control assays. A beneficial oral bacteria biofilm growth test assay comprises culturing a species of beneficial oral bacteria in media that comprises arginine and a test compound or composition and measuring the growth of the biofilm produced by beneficial oral bacteria in the beneficial oral bacteria growth test assay. A pathogenic oral bacteria biofilm growth test assay comprises culturing a species of pathogenic oral bacteria in media that comprises arginine and the test compound or composition and measuring the growth of the biofilm produced by the pathogenic oral bacteria in the pathogenic oral bacteria growth test assays. A beneficial oral bacteria biofilm growth control assay comprises culturing a species of beneficial oral bacteria in media that comprises arginine free of the test compound or composition and measuring the growth of the biofilm produced by the beneficial oral bacteria in the beneficial oral bacteria growth control assay. A pathogenic oral bacteria biofilm growth control assay comprises culturing a species of pathogenic oral bacteria in media that comprises arginine free the test compound or composition and measuring the growth of biofilm produced by the pathogenic oral bacteria in the pathogenic oral bacteria growth control assay. Total biofilm growth in each assay is assessed using crystal violet staining. Ratios are calculated as in the beneficial and pathogenic oral bacteria growth assays above.

Beneficial oral bacteria biofilm growth test assays and pathogenic oral bacteria biofilm growth test assays can include a series of such test assays using the media supplemented with 5, 10, 20, and 25 micromol/ml of arginine. Test assays can use media supplemented with at least 5 micromol/ml, at least 10 micromol/ml, at least 20 micromol/ml, at least 25 micromol/ml , about 0.05-0.1 mg/ml, about 0.1-1.0 mg/ml, about 1 mg/ml, about 2 mg/ml, about 3 mg/ml, about 4 mg/ml, about 5 mg/ml, about 6 mg/ml, about 7 mg/ml, about 8 mg/ml, about 9 mg/ml, about 10 mg/ml, about 11 mg/ml, about 12 mg/ml, about 13 mg/ml, about 14 mg/ml and/or about 15 mg/ml or arginine. Such test assays can include a series of such test assays using the media supplemented with a test compound or composition present in different concentrations. The bacterial cultures are incubated under conditions to which promote biofilm growth. The bacterial cultures can be incubated under anaerobic conditions or an environment containing 5% carbon dioxide. Total biofilm growth can be determined at one or more time points, such as for example daily for 1-4 days (i.e. at 24, 48, 72 and 96 hours). Beneficial oral bacteria used may be selected from *Streptococcus gordonii, Actinomyces viscosus, Streptococcus salivarius, Streptococcus oralis, Streptococcus mitis* and *Streptococcus sanguinis.* Pathogenic oral bacteria used may be selected from *Aggregatibacter actinomycetemcomitans, Fusobacterium nucleatum, Porphyromonas gingivalis, Prevotella intermedia, Streptococcus mutans* and *Streptococcus sobrinus.*

### EXAMPLES

### Example 1

To assess the impact of the amino acid arginine on beneficial oral bacteria and on pathogenic oral bacteria, representative bacterial species were cultured in media supplemented with 5, 10, 20, and 25 micromol/ml of arginine using a 96-well polystyrene microtiter plate. The bacterial cultures were incubated under anaerobic conditions or an environment containing 5% carbon dioxide, monitoring growth at 24 and 48 hours through optical density at a wavelength of 630 nm.

While both pathogenic and beneficial bacteria have the capacity of metabolizing arginine, the amino acid appears to have a selective activity, preferentially promoting the growth of beneficial bacteria over pathogens (Table 1).

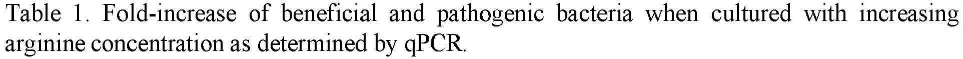

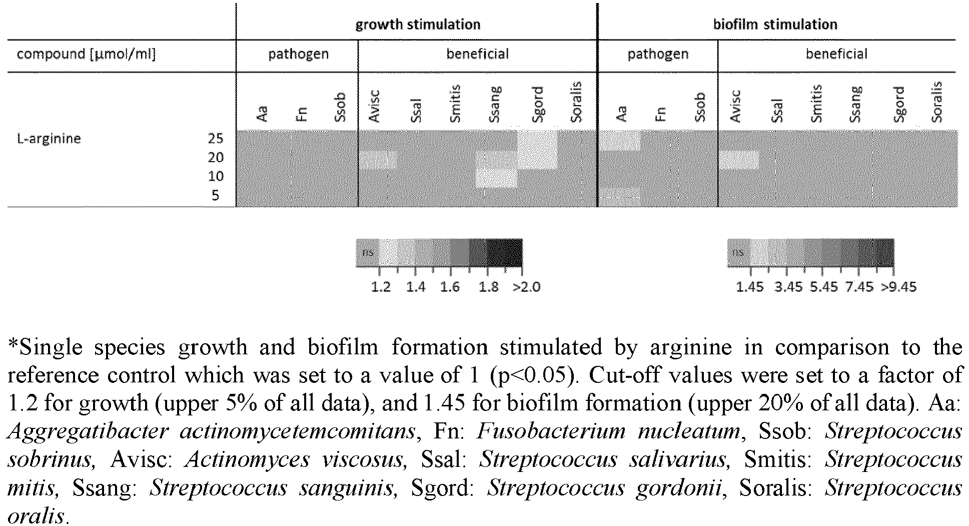

Under the described conditions, the growth of beneficial microbes (e.g. *A. viscosus, S. sanguinis,* and *S. gordonii*) was stimulated with 10-25 micromol/ml of arginine by 1.2-1.3-fold increase. Interestingly, no significant increase of pathogenic bacteria growth was observed.

### Example 2

Total biofilm growth in the presence of arginine was assessed through crystal violet staining. An increase of 1.2-fold or greater over the no substrate control was considered significant as calculated using a Student's t-test (p < 0.05).

Assessment of formed biofilms showed that while an increase in *A. actinomycetemcomitans* biofilms was observed, no significant increase in biofilm formation was observed for both pathogenic and beneficial microbes.

### Example 3

The impact of arginine in a dual-species biofilm was assessed by co-culturing one beneficial bacterial species with on pathogenic microbe in media containing 25 micromol/ml of arginine. The total beneficial-to-pathogen load in the results biofilms were determined via qPCR using DNA probes.

Using a competitive biofilm assay, the role of arginine as a selective nutrient was assessed using a dual species biofilm model (Table 2).

**Table 2. Proportional reduction of pathogenic species in a dual species biofilm post-Arginine treatment**

| | | **Pathogens** | | | | | |
|---|---|---|---|---|---|---|---|
| | | Aggregatibacter actinomycetemcomitans | Fusobacterium nucleatum | Porphyromonas gingivalis | Prevotella intermedia | Streptococcus mutans | Streptococcus sobrinus |
| | Streptococcus gordonii | 100.61% | 474.12% | -52.94% | -98.29% | -87.64% | -50.33% |
| | Actinomyces viscosus | -25.66% | 13.35% | -97.78% | -99.99% | -23.93% | -7.87% |
| | Streptoccoccus salivarius | -35.38% | -28.32% | 90.75% | 81.56% | -15.19% | -1.33% |
| | Streptococcus oralis | 268.65% | 101.73% | -79.23% | -73.78% | -3.47% | -15.77% |
| **Beneficial** | Streptococcus mitis | 73.94% | 209.31% | -96.62% | -52.64% | -0.72% | 49.29% |
| **bacteria** | Streptococcus sanguinis | 233.40% | 112.11% | -85.49% | -99.35% | -19.30% | -40.73% |

| | | | | | | | |
|---|---|---|---|---|---|---|---|
| * %reduction was calculated based on recovered beneficial vs. pathogen CFU's after treatment of biofilms with 25 µmol/ml arginine. Reduction of pathogenic bacteria is represented by a negative value expressed in percentage. | | | | | | | |

Assessment of arginine-cultured biofilms indicated an increase in the growth of beneficial bacteria while a concurrent reduction in the number of specific pathogenic bacteria was observed, in particular *P. gingivalis* and *P. intermedia* (bacteria associated with gum disease) and *S. mutans* and *S. sobrinus* (bacteria associated with caries).

### Example 4

Oral compositions that comprise arginine are disclosed in WO/2011/123123, which corresponds to US 8,652,495. In some embodiments, the oral care composition comprises an orally acceptable vehicle; zinc oxide particles; and arginine. In some such embodiments, zinc oxide particles have a median particle size of from 1 to 7 microns; in some such embodiments, the zinc oxide particles have a particle size distribution of 3 to 4 microns, a particle size distribution of 5 to 7 microns, a particle size distribution of 3 to 5 microns, a particle size distribution of 2 to 5 microns, or a particle size distribution of 2 to 4 microns. In some of these variously described embodiments, the zinc oxide particles are present in an amount of up to 5% by weight, such as from 0.5 to 2% by weight based on the total weight of the oral care composition. In some of these variously described embodiments, the zinc oxide particles the source of zinc oxide particles is selected from a powder, a nanoparticle solution; a nanoparticle suspension; a capsule; and a bead. In some of these variously described embodiments, the oral care composition further comprises at least one additional metal oxide selected from stannous oxide, titanium oxide, calcium oxide, copper oxide and iron oxide or a mixture thereof. In some of these variously described embodiments, the arginine is L-arginine. In some of these variously described embodiments, the arginine is present in an amount of up to 5% by weight, such as from 0.5 to 5% by weight, such 2.5 to 4.5% by weight as based on the total weight of the oral care composition. In some of the various embodiments, the oral composition further comprises in addition to arginine, at least one or more amino acids is selected from cysteine, leucine, isoleucine, lysine, L-lysine, alanine, asparagine, aspartate, phenylalanine, glutamate, glutamic acid, threonine, glutamine, tryptophan, glycine, valine, proline, serine, tyrosine, and histidine, and a combination of two or more thereof. In some of these variously described embodiments, the oral care composition further comprises a polymeric adherent material. In some of these variously described embodiments, the source of zinc oxide particles is a capsule, and the capsule comprises a polymeric adherent material and in some such embodiments, the polymeric adherent material comprises one or more cellulose polymers, such as embodiments in which at least one of said one or more cellulose polymers is a hydroxyalkyl cellulose polymer selected from hydroxypropylmethyl cellulose (HPMC), hydroxyethylpropyl cellulose (HEPC), hydroxybutylmethyl cellulose (HBMC), and carboxymethyl cellulose (CMC). In some of these variously described embodiments, the polymeric adherent material comprises a mixture of two hydroxyalkyl cellulose polymers having different molecular weights and the zinc oxide which is encapsulated in the mixture of two hydroxyalkyl cellulose polymers. In some of these variously described embodiments, the polymeric adherent material comprises one or more polymers selected from a poly (ethylene oxide) polymer, polyvinylpyrrolidone (PVP), polyethylene glycol (PEG)/ polypropylene glycol (PPG) copolymer, ethylene oxide (EO) - propylene oxide (PO) block copolymers, ester gum, shellac, pressure sensitive silicone adhesives, methacrylates, or mixtures thereof. In some of these variously described embodiments, the oral care composition is a dentifrice composition, such as for example, a toothpaste or a gel. In some of these variously described embodiments, the oral care composition is formulated into a form selected from a mouth rinse, a gum, a dissolvable lozenge, and a dissolvable film.

### Example 5

Oral compositions that comprise arginine are disclosed in WO 2014/088572, which corresponds to US 2015/0313813. In some embodiments the oral care composition comprises: from about 0.05 to about 5% by weight, of a zinc ion source; a fluoride ion source in an amount effective to deliver from about 500 to about 5,000 ppm fluoride, and from about 0.1 to about 10%, by weight, of arginine. In some such embodiments, the zinc ion source is selected from zinc citrate, zinc sulfate, zinc silicate, zinc lactate, zinc phosphate, zinc oxide, and combinations thereof, for example, in an amount effective to inhibit erosion. In some such embodiments, the oral composition is in the form of a dentifrice comprising an abrasive. In some such embodiments, the amount of zinc is 0.5 to 4% by weight. In some such embodiments, the compositions may further comprise one or more abrasives, one or more humectants, and one or more surfactants. In some such embodiments, the compositions may further comprise an effective amount of one or more alkali phosphate salts and/or an effective amount of one or more antibacterial agents and/or a whitening agent. In some such embodiments, the composition comprises zinc phosphate and one or more other sources of zinc ion. In some such embodiments, the pH of the composition is basic. In some such embodiments, the composition may comprise, in a silica abrasive dentifrice base: 1 to 3% zinc citrate; 1 to 8% arginine; 700 to 2000 ppm fluoride; and 2 to 8% alkali phosphate salts selected from sodium phosphate dibasic, potassium phosphate dibasic, dicalcium phosphate dihydrate, tetrasodium pyrophosphate, tetrapotassium pyrophosphate, calcium pyrophosphate, sodium tripolyphosphate, and a combination of two or more thereof.

### Example 6

Oral compositions that comprise arginine are disclosed in WO 2015/094849, which corresponds to US 2016/0338921. In some embodiments the oral care composition comprises: arginine, in free or salt form; and zinc oxide and zinc citrate. In some embodiments, the arginine is present in an amount of 0.5 weight % to 3 weight %, such as 1 weight % to 2.85 weight %, such as 1.17 weight % to 2.25 weight %, such as 1.4 weight % to 1.6 weight %, such as about 1.5 weight %, based on the total weight of the composition. In some embodiments set out above, the total concentration of zinc salts in the composition is 0.2 weight % to 5 weight %, based on the total weight of the composition. In some embodiments set out above, the molar ratio of arginine to total zinc salts is 0.05:1 to 10:1. In some embodiments set out above, the composition comprises zinc oxide in an amount of 0.5 weight % to 1.5 weight %, such as 1 weight %, and zinc citrate in an amount of 0.25 weight % to 0.75 weight %, such as 0.5 weight %, based on the total weight of the composition. In some embodiments set out above, the weight ratio of zinc oxide to zinc citrate is 1.5:1 to 4.5:1, optionally 1.5:1 to 4:1, 1.7:1 to 2.3:1, 1.9:1 to 2.1:1, or about 2:1.

### Example 7

Oral compositions that comprise arginine are disclosed in WO 2017/003844, which corresponds to US 2018/0021234. In some embodiments, the oral care composition comprises: arginine, zinc oxide and zinc citrate and a fluoride source. In some embodiments, the arginine has the L-configuration. In some embodiments, the arginine is present in an amount corresponding to 0.1% to 15%, or 0.1% to 8%, or about 5.0 wt. %, or about 8.0 wt. %, or about 1.5 wt. %, based on the total weight of the composition, the weight of the arginine acid being calculated as free form. In some embodiments, the arginine is in free form or partially or wholly salt form. In some embodiments set out above, the ratio of the amount of zinc oxide (by wt %) to zinc citrate (by wt %) is 2:1, 2.5:1, 3:1, 3.5:1 or 4:1, wherein the ratio is by wt. of the overall composition. In some embodiments, the zinc citrate is in an amount of from 0.25 to 1.0 wt % and zinc oxide may be present in an amount of from 0.75 to 1.25 wt % or the zinc citrate is in an amount of about 0.5 wt % and zinc oxide is present in an amount of about 1.0%, based on the total weight of the composition. In some embodiments set out above, the fluoride source is sodium fluoride or sodium monofluorophosphate. In some such embodiments, the sodium fluoride or sodium monofluorophosphate is from 0.1 wt. %-2 wt. % based on the total weight of the composition. In some embodiments, the sodium fluoride or sodium monofluorophosphate is a soluble fluoride salt which provides soluble fluoride in amount of 50 to 25,000 ppm fluoride, such as in an amount of about 1000 ppm-1500 ppm, for example in an amount of about 1450 ppm. In some embodiments the fluoride source is sodium fluoride in an amount about 0.32% by wt, based on the total weight of the composition. In some embodiments, the fluoride source is stannous fluoride. Some embodiments set out above further comprise a preservative selected from: benzyl alcohol, Methylisothizolinone ("MIT"), Sodium bicarbonate, sodium methyl cocoyl taurate (tauranol), lauryl alcohol, and polyphosphate. Some embodiments set out above further comprise benzyl alcohol in an amount of from 0.1-0.8% wt %, or from 0.3-0.5% wt %, or about 0.4 wt % based on the total weight of the composition. In some embodiments, the oral care composition comprises about 1.0% zinc oxide, about 0.5% zinc citrate, about 1.5% L-arginine, about 1450 ppm sodium fluoride, and optionally about benzyl alcohol 0.1 wt. % and/or about 5% small particle silica (e.g., AC43), based on the total weight of the composition. In some embodiments, the oral care composition comprises about 1.0% zinc oxide, about 0.5% zinc citrate, about 5% L-arginine, about 1450 ppm sodium fluoride, and optionally about benzyl alcohol 0.1 wt. % and/or about 5% small particle silica (e.g., AC43), based on the total weight of the composition. In some embodiments set out above, the oral care composition may comprise about 1.0% zinc oxide, about 0.5% zinc citrate, about 1.5% L-arginine, about 0.22%-0.32% sodium fluoride, about 0.5% tetrasodium pyrophosphate, and optionally about benzyl alcohol 0.1 wt. %, based on the total weight of the composition. In some embodiments set out above, the oral care composition may be any of the following oral care compositions selected from the group consisting of: a toothpaste or a dentifrice, a mouthwash or a mouth rinse, a topical oral gel, and a denture cleanser.

### Example 8

Oral compositions that comprise arginine are disclosed in WO 2017/003856. In some embodiments, the oral care composition comprises: arginine in free or salt form, zinc oxide and zinc citrate and a fluoride source comprising stannous fluoride. In some embodiments, the arginine has the L-configuration. In some embodiments, the arginine is present in an amount corresponding to 0.1% to 15%, or 0.1% to 8%, or about 5.0 wt. %, or about 8.0 wt. %, or about 1.5 wt. %, based on the total weight of the composition, the weight of the arginine acid being calculated as free form. In some embodiments, the arginine is in free form or partially or wholly in salt form. In some embodiments set out above, the ratio of the amount of zinc oxide (by wt. %) to zinc citrate (by wt. %) is 2:1, 2.5:1, 3:1, 3.5:1 or 4:1, wherein the ratio is by weight of the overall composition. In some embodiments set out above, the zinc citrate is in an amount of from 0.25 to 1.0 wt. % and zinc oxide may be present in an amount of from 0.75 to 1.25 wt. % or the zinc citrate is in an amount of about 0.5 wt. % and zinc oxide is present in an amount of about 1.0 wt. %, based on the total weight of the composition. In some embodiments set out above, the fluoride source further comprises at least one member selected from the group of: sodium fluoride, potassium fluoride, sodium monofluorophosphate, sodium fluorosilicate, ammonium fluorosilicate, amine fluoride (e.g., N'-octadecyltrimethylendiamine- N,N,N'- tris(2-ethanol)-dihydrofluoride), ammonium fluoride, titanium fluoride, hexafluorosulfate, and combinations thereof. In some embodiments set out above, the stannous fluoride is present in an amount from 0.1 wt. % to 2 wt. % based on the total weight of the composition. In some embodiments set out above, the stannous fluoride is a soluble fluoride salt which provides soluble fluoride in amount of 50 to 25,000 ppm fluoride, or about 750 -7000 ppm, or about 1000-5500 ppm, or about 5000 ppm. In some embodiments, the oral care composition comprises about 1.0% zinc oxide, about 0.5% zinc citrate, about 1.5% L-arginine, about 750 - 7000 ppm fluoride; and optionally, about 5% small particle silica (e.g., AC43), based on the total weight of the composition. In some embodiments, the oral care composition comprises about 1.0% zinc oxide, about 0.5% zinc citrate, about 750 - 7000 ppm stannous fluoride; and optionally about 39.2% glycerin based on the total weight of the composition. In some embodiments set out above, the oral care composition may comprise about 1.0% zinc oxide, about 0.5% zinc citrate, about 1.5% L-arginine, stannous fluoride, and optionally about benzyl alcohol 0.1 wt. %, based on the total weight of the composition. In some embodiments set out above, the oral care composition may be any of the following oral compositions selected from the group consisting of: a toothpaste or a dentifrice, a mouthwash or a mouth rinse, a topical oral gel, and a denture cleanser.

### Example 9

A dentifrice composition having the formula of Table A was prepared. The compositions had varying amounts of zinc oxide (varying from 0 to 2 wt%) and of amino acid (varying from 0 to 5 wt%). One example of a dentifrice composition comprised 1 wt% zinc oxide powder, the ZnO being encapsulated, at a 50 wt% loading, into a polymer film, in particular into a polymer film comprising a combination of two different molecular weight HPMC materials, comprising Methocel E5 and Methocel E50. The combined zinc oxide/ polymer film comprised 1 wt% ZnO and 1 wt% polymer film, each weight being based on the total weight of the composition. The exemplified dentifrice composition comprised 4.3 wt% L-arginine.

**Table A**

| Ingredient | % w/w | |
|---|---|---|
| Sorbitol | Q.S. | Q.S. |
| Water | 11.994 | 11.994 |
| Silica - Zeodent 105 | 10.000 | 10.000 |
| Silica - Zeodent 114 | 10.00 | 10.00 |
| Polyethylene glycol 600 | 3.00 | 3.00 |
| Silica - Zeodent 165 | 2.75 | 2.75 |
| ZnO | 0-2 | 1* |
| Sodium lauryl sulfate | 1.500 | 1.500 |
| Cocamidopropyl Betaine | 1.250 | 1.250 |
| Flavor | 1.150 | 1.150 |
| Titanium Dioxide | 0.750 | 0.750 |
| Sodium CMC - Type 7MF | 0.065 | 0.065 |
| Arginine | 0-5 | 4.3 |
| Sodium Saccharin | 0.270 | 0.270 |
| Sodium Fluoride | 0.243 | 0.243 |
| *ZnO in polymer form - 50wt% loading into polymer film comprising Methocel E5 and Methocel E50 (combined ZnO 1%/polymer -1%) | | |

### Example 10

Test dentifrices comprising 1% and 2% zinc citrate in combination with 5% arginine, 1450 ppm fluoride, and phosphates are prepared as shown in Table B:

**Table B**

| Ingredient | Zinc 1% | Zinc 2% |
|---|---|---|
| PEG600 | 3.00 | 3.00 |
| CMC-7 | 0.65 | 0.65 |
| Xanthan | 0.20 | 0.20 |
| Sorbitol | 28.4 | 27.4 |
| Glycerin | 20.0 | 20.0 |
| Saccharin | 0.30 | 0.30 |
| TSPP | 0.50 | 0.50 |
| cop Phosphate | 0.25 | 0.25 |
| dibasic Phosphate | 3.50 | 3.50 |
| Na Fluoride | 0.32 | 0.32 |
| Water | QS | QS |
| TiO2 | 0.50 | 0.50 |
| Abrasive silica | 8.00 | 8.00 |
| Thickener silica | 8.00 | 8.00 |
| L-Arginine | 5.00 | 5.00 |
| SLS | 1.50 | 1.50 |
| Brighter Flavor K91-5661 | 1.20 | 1.20 |
| Zinc Citrate | 1.00 | 2.00 |

### Example 11

Test dentifrices comprising arginine, zinc oxide, zinc citrate and a source of fluoride were prepared as shown in Tables C-G:

**Table C**

| Ingredient | Compound I |
|---|---|
| Humectants | 20.0-25.0 |
| Non-ionic surfactant | 1.0-2.0 |
| Amphoteric surfactant | 3.0-4.0 |
| Flavoring/fragrance/coloring agent | 2.0-3.0 |
| Polymers | 10.0-15.0 |
| pH adjusting agents | 1.5-3.0 |
| Precipitated Calcium Carbonate | 35 |
| Zinc citrate trihydrate | 0.5 |
| Zinc oxide | 1.0 |
| Sodium Fluoride -USP, EP | 0.32 |
| Arginine Bicarbonate | 13.86 |
| Demineralized water | QS |

**Table D**

| Ingredient | Compound A | Compound B | Compound C | Compound D |
|---|---|---|---|---|
| Humectants | 25.0-40.0 | 25.0-40.0 | 25.0-40.0 | 25.0-40.0 |
| Anionic surfactant | 1.0-3.0 | 1.0-3.0 | 1.0-3.0 | 1.0-3.0 |
| Flavoring/fragrance/coloring agent | 2.5-4.0 | 2.5-4.0 | 2.5-4.0 | 2.5-4.0 |
| Polymers | 4.0-6.0 | 4.0-6.0 | 4.0-6.0 | 4.0-6.0 |
| pH adjusting agents | 5.0-6.0 | 5.0-6.0 | 5.0-6.0 | 5.0-6.0 |
| Synthetic Amorphous Precipitated Silica | 16.00 | 21.37 | 17.92 | 7.81 |
| Alumina | 0.02 | 0.01 | 0.01 | 0.01 |
| Silica | - | - | - | 15.0 |
| Lauryl alcohol | 0.02 | 0.02 | 0.02 | 0.02 |
| Zinc citrate | 0.5 | 0.5 | 0.5 | 0.5 |
| Zinc oxide | 1.0 | 1.0 | 1.0 | 1.0 |
| Sodium Fluoride - USP, EP | 0.32 | 0.32 | 0.32 | 0.32 |
| L-Arginine Bicarbonate | 5.0 | 5.0 | 5.0 | 5.0 |
| Demineralized water | QS | QS | QS | QS |

**Table E**

| Ingredient | Compound E | Compound F | Compound G |
|---|---|---|---|
| Humectants | 25.0-40.0 | 25.0-40.0 | 25.0-40.0 |
| Anionic surfactant | 1.0-3.0 | 1.0-3.0 | 1.0-3.0 |
| Non-ionic surfactant | 0.1-1.0 | 0.1-1.0 | 0.1-1.0 |
| Amphoteric surfactant | 0.1-1.0 | 0.1-1.0 | 0.1-1.0 |
| Flavoring/fragrance/coloring agent | 4.0-6.0 | 4.0-6.0 | 4.0-6.0 |
| Polymers | 0.1-2.0 | 0.1-2.0 | 0.1-2.0 |
| pH adjusting agents | 5.0-6.0 | 5.0-6.0 | 5.0-6.0 |
| Thickener | 6.0 | 6.5 | 7.0 |
| Alumina | 0.1 | 0.1 | 0.1 |
| Synthetic Amorphous Precipitated Silica | 17.6 | 8.8 | 22.4 |
| Silica | - | 15.0 | - |
| Benzyl alcohol | 0.1 | 0.1 | 0.1 |
| Synthetic Amorphous Silica | 5.0 | 5.0 | 5.0 |
| Zinc citrate | 0.5 | 0.5 | 0.5 |
| Zinc oxide | 1.0 | 1.0 | 1.0 |
| Sodium Fluoride - USP, EP | 0.32 | 0.32 | 0.32 |
| L-Arginine Bicarbonate | 1.5 | 1.5 | 1.5 |
| Demineralized water | QS | QS | QS |

**Table F**

| Ingredient | Compound H | Compound I |
|---|---|---|
| Humectants | 45.0-55.0 | 35.045.0 |
| Abrasives | 14.0-16.0 | 9.0-11.0 |
| Anionic surfactant | 1.0-3.0 | 1.0-3.0 |
| Non-ionic surfactant | 0.1-1.0 | - |
| Amphoteric surfactant | 1.0-2.0 | - |
| Flavoring/fragrance/coloring agent | 1.0-3.0 | 2.0-4.0 |
| Polymers | 0.1-2.0 | 3.0-8.0 |
| pH adjusting agents | 0.1-2.0 | 4.0-8.0 |
| Silica Thickener | 5.0 | 5.0-10.0 |
| Benzyl alcohol | 0.1 | - |
| Zinc citrate trihydrate | 0.5 | 0.5 |
| Zinc oxide | 1.0 | 1.0 |
| Sodium Fluoride - USP, EP | 0.32 | 0.32 |
| L-Arginine | 1.5 | 5.0 |
| Demineralized water | QS | QS |

**Table G**

| Ingredient | Compound J | Compound K | Compound L |
|---|---|---|---|
| Humectants | 20.0-50.0 | 20.0-50.0 | 20.0-50.0 |
| Abrasives | 5.0-20.0 | 5.0-20.0 | 5.0-20.0 |
| Anionic surfactant | 1.0-3.0 | 1.0-3.0 | 1.0-3.0 |
| Non-ionic surfactant | 0.1-1.0 | 0.1-1.0 | 0.1-1.0 |
| Amphoteric surfactant | 0.1-2.0 | 0.1-2.0 | 0.1-2.0 |
| Flavoring/fragrance/coloring agent | 1.0-5.0 | 1.0-5.0 | 1.0-5.0 |
| Polymers | 0.1-2.0 | 0.1-2.0 | 0.1-2.0 |
| pH adjusting agents | 0.1-2.0 | 0.1-2.0 | 0.1-2.0 |
| Thickener | 6.0 | 6.5 | 7.0 |
| Dental type silica | - | - | 15.0 |
| High cleaning silica | - | 15.0 | - |
| Synthetic Abrasives | 10.0 | - | - |
| Synthetic Amorphous Silica | 5.0 | 5.0 | 5.0 |
| Benzyl alcohol | 0.4 | 0.4 | 0.4 |
| Zinc citrate trihydrate | 0.5 | 0.5 | 0.5 |
| Zinc oxide | 1.0 | 1.0 | 1.0 |
| Sodium Fluoride - USP, EP | 0.32 | 0.32 | 0.32 |
| L-Arginine | 1.5 | 1.5 | 1.5 |
| Demineralized water | QS | QS | QS |

### Example 12

Test dentifrices comprising arginine, zinc oxide, zinc citrate and stannous fluoride were prepared as shown in Table H:

**Table H**

| Ingredient | | | |
|---|---|---|---|
| Humectants | 20.0-60.0 | 20.0-50.0 | 20.0-50.0 |
| Abrasives | 10.0-40.0 | 5.0-20.0 | 5.0-20.0 |
| Anionic surfactant | 1.0-3.0 | 1.0-3.0 | 1.0-3.0 |
| Amphoteric surfactant | 0.5-1.5 | 0.1-2.0 | 0.1-2.0 |
| Flavoring/fragrance/coloring agent | 0.5-5.0 | 1.0-5.0 | 1.0-5.0 |
| Polymers | 1.0-10.0 | 0.1-2.0 | 0.1-2.0 |
| pH adjusting agents | 1.0-10.0 | 0.1-2.0 | 0.1-2.0 |
| Zinc citrate | 0.25-1.0 | 0.5 | 0.5 |
| Zinc oxide | 0.75-1.25 | 1.0 | 1.0 |
| Stannous Fluoride | 0.1-1.0 | 0.32 | 0.32 |
| L-Arginine | 0.1-10.0 | 1.5 | 1.5 |
| Demineralized water | QS | QS | QS |

## Claims

1. An oral care composition comprising arginine for use in a method of reducing total pathogenic oral bacteria load within an individual's oral cavity wherein
pathogenic oral bacteria are *Porphyromonas gingiva*/*is, Prevotella intermedia, Streptococcus mutans* and *Streptococcus sobrinus,* and
the method comprises the step of: applying 1-50 mg of arginine to the oral cavity.

2. The oral care composition for use according to claim 1, wherein the oral care composition is selected from the group consisting of: tooth paste containing arginine and an oral rinse containing arginine.

3. The oral care composition for use according to claim 1 or 2 wherein the method comprises the step of supplementing the oral cavity with an amount of arginine sufficient to obtain a concentration of at least 20-25 micromol/ml in biofilm in the oral cavity.

4. The oral care composition for use according to claim 1, wherein the method is further **characterized by** growth of beneficial oral bacteria being selectively promoted relative to growth of pathogenic oral bacteria in the individual's oral cavity; wherein the beneficial bacteria are *Streptococcus gordonii, Actinomyces viscosus, Streptococcus salivarius, Streptococcus oralis, Streptococcus mitis* and *Streptococcus sanguinis.*

5. The oral care composition for use according to claim 4, wherein the method is further **characterized by** growth of beneficial oral bacteria is selectively promoted relative to growth of pathogenic oral bacteria in biofilm in the individual's oral cavity; wherein the beneficial bacteria are *Streptococcusgordonii, Actinomyces viscosus, Streptococcus salivarius, Streptococcus oralis, Streptococcus mitis* and *Streptococcus sanguinis.*

## Patentansprüche

1. Arginin-enthaltende Mundpflegezusammensetzung zur Verwendung in einem Verfahren zur Verringerung der Gesamtbelastung mit pathogenen oralen Bakterien in der Mundhöhle eines Individuums, wobei
pathogene Mundbakterien *Porphyromonas gingivalis, Prevotella intermedia, Streptococcus mutans* und *Streptococcus sobrinus* sind, und
das Verfahren den folgenden Schritt umfasst: Anwenden von 1-50 mg Arginin in der Mundhöhle.

2. Mundpflegezusammensetzung zur Verwendung gemäß Anspruch 1, wobei die Mundpflegezusammensetzung ausgewählt ist aus der Gruppe bestehend aus: Arginin-enthaltender Zahnpasta und einer Arginin-enthaltenden Mundspülung.

3. Mundpflegezusammensetzung zur Verwendung gemäß Anspruch 1 oder 2, wobei das Verfahren den Schritt des Ergänzens der Mundhöhle mit einer Menge an Arginin umfasst, die ausreicht, um eine Konzentration von mindestens 20-25 Mikromol/ml im Biofilm in der Mundhöhle zu erhalten.

4. Mundpflegezusammensetzung zur Verwendung gemäß Anspruch 1, wobei das Verfahren weiterhin **dadurch gekennzeichnet ist, dass** das Wachstum nützlicher oraler Bakterien im Verhältnis zum Wachstum pathogener oraler Bakterien in der Mundhöhle des Individuums selektiv gefördert wird; wobei die nützlichen Bakterien *Streptococcus gordonii, Actinomyces viscosus, Streptococcus salivarius, Streptococcus oralis, Streptococcus mitis* und *Streptococcus sanguinis* sind.

5. Mundpflegezusammensetzung zur Verwendung gemäß Anspruch 4, wobei das Verfahren weiterhin **dadurch gekennzeichnet ist, dass** das Wachstum nützlicher oraler Bakterien im Verhältnis zum Wachstum pathogener oraler Bakterien im Biofilm in der Mundhöhle des Individuums selektiv gefördert wird; wobei die nützlichen Bakterien *Streptococcus gordonii, Actinomyces viscosus, Streptococcus salivarius, Streptococcus oralis, Streptococcus mitis* und *Streptococcus sanguinis* sind.

## Revendications

1. Composition de soins bucco-dentaires comprenant de l'arginine pour une utilisation dans un procédé de réduction de la charge totale de bactéries pathogènes buccales dans la cavité buccale d'un individu, dans laquelle les bactéries buccales pathogènes sont *Porphyromonas gingivalis, Prevotella intermedia, Streptococcus mutans* et *Streptococcus sobrinus,* et
le procédé comprend l'étape : d'application de 1 à 50 mg d'arginine dans la cavité buccale.

2. Composition de soins bucco-dentaires pour une utilisation selon la revendication 1, dans laquelle la composition de soins bucco-dentaires est choisie dans le groupe constitué de : une pâte dentifrice contenant de l'arginine et un bain de bouche contenant de l'arginine.

3. Composition de soins bucco-dentaires pour une utilisation selon la revendication 1 ou 2, dans laquelle le procédé comprend l'étape de complémentation de la cavité buccale avec une quantité d'arginine suffisante pour obtenir une concentration d'au moins 20 à 25 micromoles/ml dans le biofilm dans la cavité buccale.

4. Composition de soins bucco-dentaires pour une utilisation selon la revendication 1, dans laquelle le procédé est en outre **caractérisé par le fait que** la croissance de bactéries buccales bénéfiques est sélectivement favorisée par rapport à la croissance de bactéries buccales pathogènes dans la cavité buccale de l'individu ; dans laquelle les bactéries bénéfiques sont *Streptococcusgordonii, Actinomyces viscosus, Streptococcus salivarius, Streptococcus oralis, Streptococcus mitis* et *Streptococcus sanguinis.*

5. Composition de soins bucco-dentaires pour une utilisation selon la revendication 4, dans laquelle le procédé est en outre **caractérisé par le fait que** la croissance de bactéries buccales bénéfiques est sélectivement favorisée par rapport à la croissance de bactéries buccales pathogènes dans un biofilm dans la cavité buccale de l'individu ; dans laquelle les bactéries bénéfiques sont *Streptococcusgordonii, Actinomyces viscosus, Streptococcus salivarius, Streptococcus oralis, Streptococcus mitis* et *Streptococcus sanguinis.*
